# EUROPÄISCHE PATENTSCHRIFT

(11) **EP 3 389 965 B1**
(45) Veröffentlichungstag und Bekanntmachung des Hinweises auf die Patenterteilung: **09.02.2022**
(21) Anmeldenummer: 16822648.8
(22) Anmeldetag: 16.12.2016
(51) Int. Cl.: B28C 7/02, B01F 15/00, G01N 33/38

(54) **VERFAHREN ZUR BETONHERSTELLUNG**
METHOD FOR PRODUCING CONCRETE
PROCÉDÉ DE FABRICATION DE BÉTON

(30) Priorität: 17.12.2015 DE 102015225810
(43) Veröffentlichungstag der Anmeldung: 24.10.2018
(73) Patentinhaber: Universität Stuttgart, 70174 Stuttgart (DE)
(72) Erfinder: GARRECHT, Harald, 76297 Stutensee-Staffort (DE); BAUMERT, Christian, 73760 Ostfildern (DE)
(74) Vertreter: Pietruk, Claus Peter
(86) Internationale Anmeldenummer: PCT/EP2016/081379
(87) Internationale Veröffentlichungsnummer: WO 2017/103067

(56) Entgegenhaltungen:
- US-A1- 2011 029 134
- US-A1- 2012 020 180
- Lödige: "HES - High Efficiency Shovel", , 1. Juni 2015 (2015-06-01), XP055360644, Gefunden im Internet: URL:http://www.loedige.de/fileadmin/loedig e/documents/Englisch/HES_High_Efficiency_S hovel-201506.pdf [gefunden am 2017-03-31]

## Beschreibung

Die vorliegende Erfindung betrifft das oberbegrifflich Beanspruchte und bezieht sich somit auf die Herstellung von Beton mittels einer Mischvorrichtung.

Beton ist ein Baustoff, zu dessen Herstellung eine Gesteinskörnung mit einem Bindemittel vermischt wird, wobei während des Mischvorgangs Wasser sowie Betonzusatzstoffe und Betonzusatzmittel zugegeben werden, um die Betoneigenschaften zu beeinflussen.

Die Herstellung von Beton ist dabei nicht nur deswegen energieintensiv, weil Bindemittel wie Zement, dessen Herstellung große Mengen an Energie verbraucht, erforderlich sind, sondern auch, weil der Mischvorgang als solcher sehr energieaufwändig ist, besonders dort, wo moderne Betonsorten hergestellt werden sollen, beispielsweise selbstverdichtender Beton, Hochleistungsbeton (ultra high performance concrete, UHPC) usw. Als selbstverdichtend wird ein Beton verstanden, der allein durch die Schwerkraft entlüftet und in der Lage ist, eine Bewehrung zu umschließen. Nun wird bereits bei herkömmlichem Transportbeton etwa ein Sechstel des Gesamtenergieaufwandes für das Mischen benötigt; es wird einzuschätzen sein, dass moderne Hochleistungsbeton-Sorten noch deutlich mehr Energie für das Mischen benötigen als herkömmlicher Beton.

Es ist daher sinnvoll, den Mischvorgang genau zu beobachten und nur so lange zu mischen, wie es tatsächlich zur Erzielung der gewünschten Eigenschaften erforderlich ist. Zugleich soll dafür Sorge getragen werden, dass bestimmte Eigenschaften des Betons, die für den jeweiligen Einsatzzweck relevant sind, tatsächlich erreicht werden.

Es ist bereits vorgeschlagen worden, an einer Mischvorrichtung Signale zu erfassen, die für die Drehzahl der Mischwerkzeuge und das Drehmoment, mit welchem die Mischwerkzeuge angetrieben werden, indikativ sind, um im Ansprechen auf diese drehzahl- und drehmomentindikativen Signale, die an der Vorrichtung erfasst werden, Betoneigenschaften zu charakterisieren.

Eine solche Charakterisierung des Betons während der Durchmischung erlaubt es, sicherzustellen, dass geforderte Eigenschaften erreicht worden sind, oder festzulegen, dass die Mischung durch Zusätze verändert werden muss.

Eine bei selbstverdichtendem Beton besonders relevante Eigenschaft ist das Fließverhalten, welches angibt, wie der noch nicht ausgehärtete Beton unter seinem eigenen Gewicht und somit ohne Krafteinwirkung von außen fließt.

Dieses Fließverhalten ist einerseits relevant, weil das Fließen des noch nicht ausgehärteten Betons unter seinem Eigengewicht in der Praxis von besonderer Bedeutung ist. Es darf aber nicht vergessen werden, dass Beton nicht nur unter seinem Eigengewicht fließen muss, sondern dass bis zu seinem Aushärten in einer am Bau verwendeten Schalung beispielsweise auch gepumpt werden muss, was ebenfalls die Einhaltung eines bestimmten Fließverhaltens erfordert. Zudem wird einzuschätzen sein, dass sich das Verhalten des noch flüssigen Betons bei Krafteinwirkung während des Herstellprozesses nach und nach verändert.

Die Betoncharakterisierung und insbesondere die Charakterisierung des Beton-Fließverhaltens sind daher wichtig.

Eine relevante Größe bei der Beschreibung eines Fließverhaltens ist beispielsweise die Schergeschwindigkeit, welche in der Rheologie ein Maß für die mechanische Belastung darstellt, die in einer viskosen Flüssigkeit aufgrund von Strömung durch Reibungskräfte auftritt. Die mechanische Belastung kann sich aber mit der Fließgeschwindigkeit ändern. So gibt es eine Reihe von Flüssigkeiten, die überhaupt erst nach Überschreiten einer Grenzspannung zu fließen beginnen, und insoweit eine Kombination aus viskosem (fließenden) Verhalten und dem Verhalten eines elastisch verformbaren Körpers zeigen, wie dies typisch für Beton nach dem häufig herangezogenen, wenn auch nicht völlig präzisen Bingham-Hooke-Modell der Fall ist.

Deshalb erfordert z.B. eine präzise Charakterisierung des Fließverhaltens die Erstellung einer Fließkurve, um die Zusammenhänge zwischen z.B. Geschwindigkeit und Viskosität präzise zu erfassen.

Die Charakterisierung der Eigenschaften soll nun zwar einerseits präzise genug sein, um die Einhaltung der zu gewährleistenden Eigenschaften ungeachtet der in der praktischen Fertigung durch Schwankungen im Ausgangsmaterial der Mischung auftretenden Änderungen zu gewährleisten; andererseits soll aber der Durchmischungsprozess weder über Gebühr verlängert werden noch ein komplizierter manueller Eingriff erforderlich sein.

Es ist bereits bekannt, dass die Erfassung von drehzahl- und drehmomentindikativen Signalen an einer Mischvorrichtung helfen kann, Betoneigenschaften zu bestimmen. So wird in der DE 40 20 252 A1 vorgeschlagen, Eigenschaften von Gemengen durch Belastungsmessungen an Betonaufbereitungsanlagen zu ermitteln, und zwar, indem zur Bestimmung eines Verlustindexes jeweils die Leerlauf- und Belastungsdrehzahl des Mischmotors mittels eines Drehzahlgebers im Messgerät und zur Bestimmung der Belastung parallel zur Drehzahlmessung vom Mischermotor über einen Wandler die Phasenverschiebung, ebenfalls jeweils im Leerlauf und unter Belastung, von Strom und Spannung im Messgerät gemessen und danach in einem Prozessrechner ausgewertet werden.

Um einen Wasseranspruch von Bindemitteln, ihren Gemengen mit Zusatzstoffen und Zuschlägen als Grundlage für die Projektierung und Herstellung von Mörteln und Betonen in einem Mischer durch Messung der Belastung des Mischerantriebes bei kontinuierlicher Wasserzugabe zu ermitteln, wurden folgende Schritte in der DE 1 00 54 823 C1 vorgeschlagen: Füllen des Mischers mit Bindemittel und ihren Gemengen mit Zusatzstoffen und Zuschlägen in definierter Menge; kontinuierliche Wasserzugabe in den Mischer; Erfassung der ansteigenden Belastung des Mischerantriebes; Auswertung der sich einstellenden Belastungskurve mit Glättung der Belastungskurve, wobei die Glättung erst nach Beendigung des Messvorgangs vorgenommen wird, und exakte Ausweisung der zum Belastungsmaximum gehörenden Wassermenge als Wasseranspruch.

Aus der DE 1 95 03 028 A1 ist ein Verfahren mit einer Vorrichtung zum Messen des Fließverhaltens grobkörniger Stoffgemenge bekannt, wobei ausgeführt ist, dass der Verlauf der Fließkurve bei Frischbetonen in der Nähe der Ordinate annähernd linear ist. Deswegen soll aus zwei Messwerten der Verlauf der ganzen Fließkurve über die Geschwindigkeit vergleichsweise sicher anzugeben sein, wenn der Rührwiderstand als nahezu proportional mit der Geschwindig-Aus der EP 1 550 535 A1 ist ein Verfahren zur Herstellung einer Betonmasse in einem Mischer bekannt, wobei das eingetragene Drehmoment und/oder der Leistungsverbrauch bei zumindest zwei Mischgeschwindigkeiten erfasst werden soll(en) und basierend auf den Messresultaten die Eigenschaften der Betonmasse bestimmt werden sollen. Auf die zur gleichen Patentfamilie gehörende EP 1 550 868 B1 sei hingewiesen.

Auch gemäß der GB 2 147 215 A wird ein Drehmoment bestimmt, um über die Zugabe von Stoffen zu entscheiden.

Die US 4 281 288 A schlägt vor, die Genauigkeit einer Messung ungeachtet von Schlupf zu erhöhen, indem bei einem Induktionsmotor in einem Bereich gemessen wird, wo ein ungefähr linearer Zusammenhang zwischen Drehmoment und Drehgeschwindigkeit gegeben sein soll.

Aus der US 2011/0029134 A1 ist ein Verfahren zur Herstellung von Beton und eine dazugehörende Mischvorrichtung gemäß dem Oberbegriff des Anspruchs 1, bzw. Anspruch 12 bekannt, insbesondere ist es hieraus bekannt, die Thixotropie zu überwachen, indem eine reversible, zeitabhängige Verringerung der Viskosität gemessen wird, die beim Mischen von Beton auftritt.

Aus der US 2012/0020180 A1 ist ein Verfahren bekannt, um Information über Menge und Charakteristik zementhaltigen Materials in einer sich drehenden Mischtrommel zu bestimmen. Dabei soll eine Zeitreihe von Werten, die auf die zum Drehen einer Mischertrommel erforderliche Energie bezogen sind, bestimmt und mit einer bekannten Reihe verglichen werden, um dann die Rheologie einzustellen.

Es wäre vorteilhaft, das Fließverhalten von z.B. selbstverdichtendem Beton besser charakterisieren zu können, als dies im Stand der Technik möglich ist, insbesondere dahingehend, dass eine Messung in einer zur Betonproduktion für Baustellen verwendbaren Mischvorrichtung erfassbar wird, und zwar bevorzugt schnell und zumindest so genau, dass die Einhaltung gewünschter Fließverhaltenseigenschaften gewährleistet werden kann, selbst wenn keine absoluten Messwerte angegeben werden können. Es wäre alternativ und/oder zusätzlich auch vorteilhaft, zumindest eine Betoneigenschaft schnell, einfach und präzise zu messen. Es wäre alternativ und/oder zusätzlich auch vorteilhaft, Maschinenzeiten für die Durchmischung zu verkürzen, weil Maschinenzeiten teuer sind. Es wäre alternativ und/oder zusätzlich auch vorteilhaft, mit kleineren Mischern größere Beton-Volumina erzeugen zu können. Es wäre alternativ und/oder zusätzlich auch vorteilhaft, eine hohe Beton-Qualität gewährleisten zu können. Es wäre alternativ und/oder zusätzlich auch vorteilhaft, Beton mit niedrigerem Energieaufwand erzeugen zu können.

Wünschenswert wäre, zumindest einige der oben genannten Vorteile zumindest partiell zu erzielen.

Die Aufgabe der vorliegenden Erfindung besteht darin, Neues für die gewerbliche Anwendung bereitzustellen und insbesondere eine bessere Betonherstellung zu ermöglichen.

Die Lösung dieser Aufgabe wird in den unabhängigen Ansprüchen angegeben. Bevorzugte Ausführungsformen finden sich in den Unteransprüchen.

Gemäß einem ersten Grundgedanken der Erfindung wird somit vorgeschlagen, dass bei einem Verfahren zur Herstellung von Beton mittels einer Mischvorrichtung, wobei drehzahl- und drehmomentindikative Signale an der Vorrichtung erfasst und Betoneigenschaften im Ansprechen darauf bestimmt werden, vorgesehen ist, dass bei der Bestimmung der Betoneigenschaften das Losbrechverhalten der Vorrichtung berücksichtigt wird, dass bei der Bestimmung der Betoneigenschaften das Losbrechverhalten der Vorrichtung berücksichtigt wird, indem das zum Losbrechen erforderliche Drehmoment ermittelt wird und von den zu Erzielung einer bestimmten Drehzahl erforderlichen Drehmoment abgezogen wird.

Dies erlaubt eine sehr genaue Charakterisierung eines Mischprozesses bzw. eine exakte Kompensation von Schwankungen in den Betoneigenschaften, die durch Variationen der Ausgangsmaterial-Zusammensetzung, schwankende Feuchten im Ausgangsmaterial usw. verursacht werden. Die genaue Charakterisierung erlaubt wiederum eine Kompensation der Schwankungen zwecks Einhaltung von Grenzwerten. Dies führt, obwohl keine für rheologische Messungen definierten Oberflächen und dergleichen vorhanden sein müssen, zu einer massiven Verbesserung der Reproduzierbarkeit von gewünschten Betoneigenschaften und erhöht so die Betonqualität.

Es ist dabei zu beachten, dass das erfindungsgemäße Verfahren keine Absolutwerte für die betrachteten Betoneigenschaften liefern muss, sondern lediglich die Zusammenhänge zwischen tatsächlich erhaltenen Eigenschaften und den an der Maschine erfassten Größen so genau charakterisiert werden braucht, dass eine hinreichende Sicherheit der Prozessführung gewährleistet ist. Dabei ist es für Zwecke der Erfindung unerheblich, dass jeder selbstverdichtende Beton sein eigenes Mischverhalten besitzt und demgemäß das Fließverhalten unterschiedlicher selbstverdichtender Betonsorten stark voneinander abweicht.

Bestimmt werden kann als Betoneigenschaft z.B. die Fließgrenze und/oder die Viskosität, bevorzugt beides. Es sei darauf hingewiesen, dass gerade im Bereich von Hochleistungsbeton ganz erhebliche Schwankungen von rheologisch relevanten Parametern zu beobachten sein werden und dass die Einhaltung von Grenzwerten für solche rheologisch relevanten Parameter von fundamentaler Bedeutung für die Eigenschaften eines mit dem Beton zu bauenden Bauwerkes sein kann. Zudem wird einsichtig sein, dass das Verhalten von zum Beispiel Scherrate gegen Scherspannung (oder Drehmoment gegen Drehzahl; Viskosität gegen Fließgeschwindigkeit und so weiter) bei typischen Hochleistungsbetonsorten alinear sein kann. Typisch tritt dabei eine Scherverdickung ein, das heißt, es wird ein Ansteigen der Viskosität mit zunehmender Scherrate zu erwarten sein.

Es wird einzuschätzen sein, dass auch zum Erhalt einer noch brauchbaren Betonmischung noch Variationen zuzulassen sind. So wird typisch ein "Fenster" von Viskosität und Fließgrenze gegeben sein, innerhalb von welchem ein Beton liegen muss, um sich als brauchbar zu qualifizieren.

Dabei kann die Erfindung ohne weiteres an einem für Anwendungen auf Baustellen und dergl. brauchbaren Mischer angewendet werden, da gewährleistet wird, dass das Gesamtsystem aus Umrichter, Motor, Getriebe und Mischer bzw. Mischerwellen betrachtet wird.

Die Erfindung berücksichtigt somit den Antriebszug Umrichter - Motor - Getriebe- Welle-Mischer. Die Drehzahlen des Motors können wie üblich mit dem Getriebe untersetzt werden; angestrebt werden Drehzahlen von beispielsweise ca. 30 U/min.

Als Losbrech-Kraft wird allgemein jene Kraft verstanden, die aufgewendet werden muss, um die Haftreibung im Ruhezustand zu überwinden und einen Gleitzustand einzuleiten, für den die Gleitreibung maßgeblich ist. Es wird einsichtig sein, dass bei Drehantrieben an Stelle der Losbrech-Kraft ein Losbrech-Drehmoment tritt. Entsprechend ist der Begriff des Losbrech-Verhaltens zu verstehen, wobei darauf hingewiesen wird, dass bei einer technischen Vorrichtung wie einem Mischer die Losbrech-Kraft bzw. das Losbrech-Drehmoment keinesfalls dauerhaft konstant sein braucht.

Indem das Losbrech-Verhalten berücksichtigt wird, können Messungen bei sehr niedrigen Drehzahlen und demgemäß auch entsprechend niedrigem Drehmoment durchgeführt werden, wobei dann Messungen an derartigen Maschinen dank der Erfindung kaum durch Losbrech-Effekte beeinträchtigt sind. Es muss zu niedrigen Werten nicht mehr grob extrapoliert werden, sondern es sind jetzt genaue Messungen möglich, die auch unter kritischen Bedingungen eine Fließgrenzen-Bestimmung zulassen.

Der Zusammenhang zwischen Drehzahlen und Drehmoment kann somit auch im unteren Bereich wesentlich präziser erfasst werden als bislang möglich und die Messungen werden dementsprechend deutlich aussagekräftiger. Dies ist von Vorteil, um den Beton besser charakterisieren zu können, weil einerseits der Zusammenhang zwischen Drehmoment und Drehzahlen bei der Herstellung von Betonmischungen keinesfalls linear ist und andererseits die bei langsamen Bewegungen erhaltenen Messwerte besonders aussagekräftig sind.

Das Verfahren ist insofern besonders vorteilhaft, weil es vergleichsweise einfach ist, für die Drehzahl indikative Signale und für das Drehmoment indikative Signale an einer Mischvorrichtung selbst zu erfassen. Es sind also, eventuell abgesehen von den eigentlichen Sensoren, praktisch keine Veränderungen an der Vorrichtung erforderlich und es kann der Beton durch Messungen charakterisiert werden, die durchgeführt werden, während der Beton sich noch in einer Mischvorrichtung befindet, ohne dass ein Umfüllen und so weiter erforderlich wäre. Es können dabei Sensoren vorgesehen sein, wie Drehmomentgeber oder Drehzahlmesser; gleichwohl ist es auch möglich, das Drehmoment, welches ein Elektromotor ausübt, anhand elektrischer Kenngrößen zu bestimmen wie unmittelbar die Motorerregung beeinflussende Größen, etwa Stromstärke und (Umrichte-)Frequenz.

Dabei ist zu beachten, dass zwar eine -möglichst exakte- Messung mittels Drehmomentgebern an der Welle o. ä. messtechnisch vorteilhaft wäre, aber in der Praxis eher mit Problemen bei einer solchen Variante zu rechnen ist und die Bestimmung anhand von die Motorerregung beeinflussenden Größen, etwa Stromstärke und (Umrichte-)Frequenz, auch bei Forderung nach enger Qualitätskontrolle für den herzustellenden Beton im Regelfall völlig ausreichen wird.

Die Messungen können zudem, wie noch ersichtlich werden wird, schnell durchgeführt werden. Betoncharakterisierende Messungen brauchen den eigentlichen Mischvorgang praktisch nicht zu verzögern, zumal die Möglichkeit besteht, den Durchmischungs-Vorgang sofort und unmittelbar fortzusetzen, falls das Messergebnis auf noch unzureichende Durchmischung deuten sollte oder darauf, dass die gewünschten Betoneigenschaften noch nicht erreicht wurden. Es kann insofern nicht nur der eigentliche Mischvorgang fortgesetzt werden, sondern es können auch eigenschaftsverändernde Zusätze zugefügt werden.

Es wird dann gefragt, wie stark bei typischen Gemischen die Unterschiede zwischen "brauchbarem" Beton und nicht brauchbarem Beton tatsächlich sind, etwa weil sich die Umgebungstemperaturen ändern, die Feuchte von bei der Herstellung verwendeten Materialien schwankt, so dass die Gesamtwassermenge variiert, und so weiter.

Es ist vorteilhaft, wenn ein solches Verfahren vor allem zur Herstellung von Ultrahochleistungsbeton und/oder selbstverdichtendem Beton vorgesehen wird.

Gerade im Bereich von Ultrahochleistungsbeton und selbstverdichtenden Betonen ist es besonders bedeutsam, das Mischverfahren sehr gut kontrollieren zu können. Einerseits erfordert (Ultra-) Hochleistungsbeton in besonderem Maße eine vollständige und gründliche Durchmischung aller Bestandteile, andererseits ist die Durchmischung im Regelfall besonders energieaufwändig, so dass der Durchmischungsprozess einen erheblichen Kostenfaktor darstellt, zumal eine überlange Durchmischung auch zu erhöhten Maschinenzeiten beiträgt und aus diesem Grund unerwünscht ist. Für selbstverdichtenden Beton ist vorteilhaft, dass dessen Fließverhalten durch die Erfindung präziser charakterisiert werden kann.

Es ist möglich und bevorzugt, dass bei Ausführung des Verfahrens insbesondere zur Herstellung von (Ultra-)Hochleistungsbeton und/oder selbstverdichtendem Beton je Charge ein Volumen aus der Gruppe von größer 100 Liter, größer 200 Liter, größer 500 Liter, größer 1000 Liter und/oder bevorzugt in einer festen oder transportablen Mischvorrichtung erzeugt wird. Es kann eine Durchmischung mit mehreren Antriebsmotoren erfolgen, wenn das erfindungsgemäße Verfahren implementiert wird.

Die Erfindung kann also bereits mit sehr kleinen Mischern erfolgreich eingesetzt werden und erlaubt auch bei diesen eine hinreichend präzise Betoncharakterisierung. Die Verwendung mit Chargen, die ein Volumen größer 100 Liter haben, zeigt, dass eine Mischung auch deutlich oberhalb eines Technikumsmaßstab sehr gut kontrolliert werden kann und dort die Erfindung gut eingesetzt werden kann. Die Verwendung mit Größen wenig über 200 Liter ist dort vorteilhaft, wo nur vergleichsweise wenig Beton verbaut werden muss. Die Verwendung von Mischervolumina größer 500 Liter bzw. >1000 Liter erlaubt auch die Anwendung auf Großbaustellen. Hierbei ist zu beachten, dass durch das Verfahren die Mischzeiten deutlich reduziert werden können, weil ein Mischen einer Dauer länger als unbedingt notwendig nur aus dem Grund, eine gewisse "Reserve" gegenüber einer unzureichenden Mischung zu haben, entfällt. Da zudem besonders hohe Antriebsleistungen verwendbar sind, können die Chargen jeweils sehr schnell erzeugt werden.

Die Erzeugung von Chargen um 500 Liter oder darüber ist insofern für typische Arbeitsabläufe auf Baustellen oder im Fertigteilwerk insoweit im Regelfall völlig ausreichend und es sei angemerkt, dass die Erfindung auch bei den entsprechend großen Antrieben von Mischmaschinen größer 500 Liter Chargenvolumen oder größer 1000 Liter Chargenvolumen dank der erfindungsgemäßen Berücksichtigung des Losbrechverhaltens ohne weiteres verwendbar ist.

Es ist möglich und bevorzugt, dass bei einem Verfahren der Erfindung vorgesehen ist, dass als Mischvorrichtung einer aus der Gruppe Ringtrogmischer, Tellermischer, Planetenmischer, Einzel- oder Doppelwellenmischer verwendet wird, bevorzugt mit einem Getriebe aus der Gruppe Planetengetriebe, Schneckengetriebe und/oder Riemenscheibe zwischen Antriebswellen und es kann die Erfindung bei Verwendung von einem oder mehreren Antriebsmotoren implementiert werden. Gemein ist diesen Mischern, dass es sich dabei um Mischer handelt, die das Mischgut vollständig oder doch zumindest nahezu vollständig durch entweder ein Mischwerkzeug oder aber durch mehrere, miteinander aber im mechanischen Verbund befindliche Gruppen von Mischwerkzeugen bewegen. Die Verwendung eines Mischer mit nur einem Mischwerkzeug bzw. einer einzelnen, im Verbund beweglichen Gruppe von Mischwerkzeugen wird, wie einsichtig ist, die Bestimmung des Losbrechmomentes erleichtern. Dies gilt insoweit auch für Doppelwellenmischer, die zwar gegebenenfalls in weniger bevorzugten Varianten einzeln angetriebene Wellen aufweisen könnten, die aber gleichwohl selbst dann ein für beide Wellen typisch praktisch gleiches Losbrechverhalten zeigen werden. Es sei zudem bezüglich der Planetenmischer erwähnt, dass selbst für diese eine Anwendung des erfindungsgemäßen Verfahrens möglich ist, und zwar völlig ungeachtet der bei einem Planetenmischer durch die beiden überlagerten Bewegungen bedingten Schwankungen in der Leistungsaufnahme.

Zunächst ist es also für die Erfindung sowohl möglich, eine Vielzahl von Mischern, insbesondere Einzelwellen-Mischer oder Doppelwellen-Mischer zu verwenden, auch wenn diese ein womöglich unterschiedliches Losbrech-Verhalten besitzen sollten; durch die Berücksichtigung desselben ist es möglich, eine Entscheidung zwischen z.B. Einzel- oder Doppelwellen-Mischer im Hinblick auf andere Kriterien zu treffen und gleichwohl eine streng kontrollierte Durchmischung gewährleisten zu können. Wo, wie häufig der Fall, ein Doppelwellenmischer bevorzugt wird, können entweder beide Wellen mit einem jeweils eigenen zugeordneten Motor angetrieben werden oder aber es wird, was bevorzugt ist, ein für beide Wellen gemeinsam verwendeter Motor eingesetzt. Dies ist schon deshalb regelmäßig zu bevorzugen, weil es den Aufbau vereinfacht.

Auch die Getriebewahl ist durch das erfindungsgemäße Verfahren nicht eingeschränkt. Es wird vielmehr einsichtig sein, dass die Wahl eines Getriebes zwar das Losbrech-Verhalten beeinflussen kann, dass aber gerade durch die erfindungsgemäße Berücksichtigung des Losbrechverhaltens Wahlfreiheiten bestehen. Die explizit genannten Getriebearten sind deshalb exemplarisch ausgewählt, weil sie bei herkömmlichen Mischvorrichtungen besondere Bedeutung besitzen und oftmals verwendet werden. Die angegebene Auswahl muss aber nicht einschränkend verstanden werden. Es sei angemerkt, dass bei einigen Getrieben zum Teil ein Spiel auftreten wird. Ungeachtet dieses Spiels, das dazu führt, dass eine Drehung ein Stück weit möglich ist, bevor ein Antrieb auf Widerstand stößt, kann aber die Messung ohne weiteres durchgeführt werden.

Es ist möglich und bevorzugt, dass bei einem Verfahren der Erfindung vorgesehen ist, dass zur Messung drehzahl- und drehmomentindikativer Signale ein Drehzahlmessgeber an einem von Mischerwelle, Getriebewelle und/oder Antriebsmotor vorgesehen ist, und drehmomentindikative Signale vor oder nach einem Getriebe erfasst werden, insbesondere durch Erfassung einer eingespeisten elektrischen Antriebsleistung für den Wellenantrieb.

Die Drehzahlmessung kann auf unterschiedliche Weise erfolgen. Ein typischer Antriebszug für einen Mischer umfasst mindestens eine Mischerwelle, bei Doppelwellenmischern offensichtlich zwei Wellen. Mischer werden demnach wenigstens eine Getriebewelle und wenigstens einen Antriebsmotor aufweisen, ohne dass die Erfindung aber auf eine Maschine mit nur einem solchen Antriebszug begrenzt sei. So können bei Doppelwellenmischern evtl. auch zwei getrennte Antriebsmotoren vorgesehen sein usw.

Die Drehzahlenmessung kann nun an einer oder mehreren Stellen innerhalb des Antriebszugs erfolgen, wie am Getriebe, am Antrieb oder anderen Stellen; gegebenenfalls wird es ohne weiteres möglich sein, die entfernt von der Mischerwelle erfassten Geschwindigkeiten bzw. Drehzahlen umzurechnen in die tatsächliche Drehung des Mischers, präziser der Mischerwelle oder seiner Werkzeuge.

Es ist möglich, mehrere Drehzahlmessgeber zu verwenden, etwa, um unter besonders rauen Bedingungen eine Funktion vollumfänglich zu gewährleisten. Auch kann das Drehmoment vor und/oder nach einem Getriebe erfasst werden, wobei darauf hingewiesen sei, dass das Drehmoment besonders einfach durch Bestimmung von elektrischen Kenngrößen wie der elektrischen Antriebsleistung für den Wellenantrieb erfasst werden kann. Die Bestimmung der Drehzahl bzw. von drehmomentindikativen Signalen für die Implementierung des erfindungsgemäßen Verfahrens erfordert somit auf Sensorseite keinen, jedenfalls keinen signifikanten Aufwand. Wie ersichtlich sein wird, kann das Losbrech- Verhalten sehr einfach durch per se herkömmliche Sensoren erfasst werden und es sind allenfalls geringe Anpassungen an einer Steuerung und/oder Prozessrechnereinheit erforderlich, so dass auch eine Nachrüstung existierender Anlagen vergleichsweise einfach durchführbar ist.

Es ist möglich und bevorzugt, dass bei einem Verfahren der Erfindung vorgesehen ist, dass die drehzahl- und drehmomentindikativen Signale an zumindest einem von Getriebe, Motor und/oder Mischerwellen erfasst und/oder die Erfassung die Digitalisierung, Konditionierung und Einspeisung der digitalisierten und evtl. konditionierten drehzahl- und drehmomentindikativen Signale in eine Prozessrechnereinheit umfasst.

Die Signalverarbeitung kann somit und wird auch bevorzugt digital erfolgen. Um eine ordnungsgemäße Signalverarbeitung zu ermöglichen, können die Signale auch zuvor konditioniert werden, also Sensorsignale wie erforderlich verstärkt, in der Impedanz angepasst, analog hoch-, tief- oder bandpassgefiltert, evtl. auch analog integriert oder differenziert werden und dergleichen, um erst danach dann einer Analog-Digital-Wandlung unterworfen zu werden. Dass digitale Signalkonditionierung, z.B. zwecks Entrauschen, Filterung, Normierung usw. aber auch möglich ist, sei erwähnt.

Es ist möglich und bevorzugt, dass bei einem Verfahren der Erfindung vorgesehen ist, dass als Betoneigenschaft das Fließverhalten des Betons bestimmt wird.

Es ist möglich und bevorzugt, dass bei einem Verfahren der Erfindung vorgesehen ist, dass die Erfassung der drehzahl- und drehmomentindikativen Signale durch Messung einer Reihe von wenigstens 2, bevorzugt 3, besonders bevorzugt wenigstens 5 Messwerten erfolgt, wobei die Messwerte erfasst werden bei einem aus der Gruppe bis 0,25 x die maximale Drehzahl während der Durchmischung; bis 0,25 x die mittlere Drehzahl über die Zeit während des Mischvorganges; bis 0,4 x die maximale Drehzahl, bis 0,4 x die mittlere Drehzahl über der Zeit während des Mischvorgangs; und dass aus der Reihe von 2, 3, beziehungsweise 5 Messwerten eine Interpolation gegen N = 0 (d.h. Drehzahl Null) erfolgt und/oder dass die Erfassung der drehzahl- und drehmomentindikativen Signale an der Vorrichtung intermittierend zum Mischvorgang erfasst werden, insbesondere bei einer Scherbelastung von zumindest 10 % unterhalb von jener, die beim Mischen und/oder Pumpen der Betonmischung auftritt, besonders bevorzugt bis zu einer Scherbelastung von maximal 25% von jener, die beim Mischen der Betonmischung (maximal) auftritt; insbesondere bevorzugt bis zu einer Scherbelastung von maximal 15% von jener, die beim Mischen der Betonmischung (maximal) auftritt und in weiter bevorzugter Ausgestaltung sogar nur 10% von jener, die beim Mischen der Betonmischung (maximal) auftritt.

Prinzipiell können mit der Erfindung unterschiedliche Eigenschaften des Betons charakterisiert werden und die Einhaltung unterschiedlicher Betoncharakteristika durch das Verfahren gewährleistet werden. Dazu kann ausgenutzt werden, dass etliche Eigenschaften eines fertigen Betons stark mit dem Verhalten der ihm zugrunde liegenden Mischung korrelieren. Besonders bevorzugt ist es, als Beton-Eigenschaft das Fließverhalten des Betons zu bestimmen. Dies erfordert nur einen geringen Aufwand, weil das Fließverhalten erfindungsgemäß leicht und genau ermittelt werden kann.

Wenn das Fließverhalten exakt bestimmt werden soll, ist es vorteilhaft, etliche Werte, bevorzugt wenigstens 3, besser wenigstens 5 Messwerte bei sehr niedrigen Scherraten zu bestimmen, also unterhalb von z.B. 25% jener Scherrate, die beim Mischen der Betonmischung (im Mittel bzw. maximal) auftritt. Die Bestimmung mehrerer Werte unterhalb von 25% der erwähnten Scherrate erlaubt es, im unteren Scherratenbereich auch Alinearitäten gut zu erfassen. Werden wenigstens 5 Messwerte bei maximal 15 % von jener Scherrate erfasst, die beim Mischen der Betonmischung (im Mittel bzw. maximal) auftritt, wird eine noch präzisere Messung erhalten, in der sich auch kleinere Alineariäten deutlicher zeigen und somit besser charakterisiert werden können. Noch besser wird eine Interpolation, wenn recht viele Messwerte wie 3 oder besser wenigstens 5 bis oder unterhalb von 10% jener Scherrate erfasst werden. Dadurch werden nämlich zugleich viele Messungen bei vergleichsweise ähnlichen Bedingungen durchgeführt und Effekte wie Temperaturanstieg im Getriebe durch höhere Last usw. in diesem Bereich werden sich weniger stark in Variationen des einschlägigen Losbrechmomentes zwischen den Einzelwerten auswirken. Dieser Effekt ist auch vorteilhaft, wenn nur wenige Messwerte in einem kleinen Scherratenbereich aufgenommen werden.

Es sei erwähnt, dass es verschiedene Möglichkeiten gibt, das Losbrech-Verhalten zu berücksichtigen. Es ist möglich und bevorzugt, dass bei einem Verfahren der Erfindung vorgesehen ist, zur Erfassung der für Drehzahl bzw. Drehmoment indikativen Signale eine Reihe von wenigstens 2, bevorzugt 3, besonders bevorzugt wenigstens 5 Messwerten aufzunehmen. Diese sollten erfasst werden bei einem aus der Gruppe von bis zu 0, 25 mal die maximale Drehzahl, bei welcher die Durchmischung erfolgt, bzw. bis zu 0,25 mal die mittlere Drehzahl über die Zeit während des Vorganges, bzw. bis 0,4 mal die maximale Drehzahl, bei welcher die Durchmischung erfolgt, bzw. bis zu 0,4 mal die mittlere Drehzahl über der Zeit während des Mischvorgangs. Dabei kann mit der Reihe von 2, 3 bzw. mindestens 5 Messwerten eine Interpolation gegen eine Drehzahl von Null erfolgen und/oder die für Drehzahlen bzw. Drehmomente indikativen Signale an der Vorrichtung intermittierend zum Mischvorgang erfasst werden, insbesondere bei einer Scherbelastung unterhalb jener, die beim Durchmischen oder Pumpen der Betonmischung auftritt, bevorzugt bis zu einer Scherbelastung von zumindest 10 % unterhalb jener, die beim Durchmischen der Betonmischung (maximal) auftritt.

Es werden also bei langsam drehender Mischvorrichtung mehrere Messwerte aufgenommen. Die Aufnahme von wenigstens 2 Messwerten bei niedrigen Drehzahlen erlaubt es, eine lineare Interpolation vorzunehmen. Obgleich von Betonmischungen kein linearer Drehzahl - Drehmoment-Zusammenhang zu erwarten ist, wird zumindest bei hinreichend niedrigen Drehzahlen und einem engen Drehzahlbereich eine noch ausreichende Linearität gegeben sein. Deswegen ist es besonders bevorzugt, falls lediglich 2 Messungen im sehr niedrigen Drehzahlbereich durchgeführt werden, dass diese 2 Messungen unterhalb der Hälfte der maximalen Drehzahl liegen, bevorzugt unter 0,3 mal der maximalen, noch mehr bevorzugt unterhalb 0,3 mal der mittleren Drehzahl, insbesondere bevorzugt nicht oberhalb 0,2 mal der maximalen Drehzahl und besonders bevorzugt unterhalb 0,2 mal der mittleren Drehzahl. Es finden entsprechend bevorzugt mehrere Messungen, insbesondere auch bevorzugt mindestens 3, weiter bevorzugt mindestens 5 Messungen im Bereich bis 20% der Maximallast, bevorzugt im Bereich bis 10% der Maximallast statt.

Es lässt sich allerdings ohne großen Aufwand realisieren, wenigstens drei Messungen bei niedrigen Drehzahlen durchzuführen. Eine Messung bei drei unterschiedlichen niedrigen Drehzahlen hat den Vorteil, dass bereits eine zumindest grobe Kontrolle dahingehend vorgenommen werden kann, ob ein hochgradig alineares Verhalten vorliegt, wobei auch ein Erkennen von großen Messungenauigkeiten möglich ist. Dies wiederum führt dazu, dass gegebenenfalls zusätzliche Messwerte aufgenommen werden könnten, falls eine zu große Alinearität beobachtet wird und/oder unerklärliche Abweichungen auftreten. Auch bei drei Messungen ist es bevorzugt, dass diese 3 Messungen bei niedriger Drehzahl unterhalb der halben maximalen Drehzahl oder der halben mittleren Drehzahl, gemittelt über die Zeit des Mischvorgangs, liegen werden.

Es sei angemerkt, dass auch nicht zwingend ein linearer Zusammenhang angenommen werden muss, selbst wenn nur wenig Messwerte vorliegen. Vielmehr kann etwa eine Interpolation auch alineare Zusammenhänge unterstellen; es kann etwa an einem Arbeitstag oder für die Verarbeitung einer gegebenen Menge gleicher Ausgangsrohstoffe zunächst eine Messung mit recht vielen Messwerten bei niedrigen Drehzahlen erfolgen, ein erwarteter Kurvenverlauf nach einer ausreichenden Mischzeit bestimmt werden und dann die später folgenden Messungen an einen so ermittelten linearen Zusammenhang angepasst werden.

Während dies per se zwar möglich ist, besteht allerdings im Regelfall keine besonderen Notwendigkeit dafür, da die Aufnahme von Messwerten bei gegebenen niedrigen Drehzahlen selbst wenig aufwändig ist, so dass ohne weiteres eine größere Zahl von Messwerten bei niedrigen (einander unterschiedlichen) Drehzahlen erfasst werden können; besonders bevorzugt ist es daher, wenn wenigstens 5 Messwerte bei niedrigen Drehzahlen erfasst werden. Dies erlaubt eine gute Bestimmung des Kurvenverlaufs, Fehlerkorrekturen und eine gute Extrapolation auf Drehzahl Null.

Es sei darauf hingewiesen, dass im Laufe eines gegebenen Misch-Vorganges bei festem eingebrachten Drehmoment die Drehzahl variieren kann, und zwar auch dann, wenn die zur eigentlichen erfindungsgemäßen Messung erforderlichen Bestimmungen für drehzahl bzw. drehmomentindikative Signale bei niedrigen Drehzahlen außer Acht gelassen werden. Es wird einzuschätzen sein, dass bestimmte Gesteinskörnungen, Zusatzstoffe und Zusatzmittel zu unterschiedlichen Zeiten zugegeben werden während des Mischvorgangs und dabei die Einmischzeiten und Drehzahlen variieren können. Die Erfindung kann dessen ungeachtet gut verwendet werden.

Es ist möglich und bevorzugt, dass bei einem Verfahren der Erfindung vorgesehen ist, dass zur Bestimmung der drehzahl- und drehmomentindikativen Signale an der Vorrichtung die Drehgeschwindigkeit stufenweise erhöht wird, abgewartet wird, bis die Drehzahl sich stabilisiert hat und eine Plateauphase für die Drehgeschwindigkeit erreicht ist. Es ist im Übrigen möglich, eine Messung im sehr niedrigen Drehzahlbereich durchzuführen. So ist es oft ohne weiteres möglich, ab Drehzahl 0 zu messen, sofern der Elektromotor in der Lage ist, eine Position gegen Last zu halten, was im Regelfall gegeben ist.

Eine Messung kann im Wechsel mit dem eigentlich Mischvorgang erfolgen, d.h. der eigentliche Mischvorgang kann für die Durchführung von Messungen unterbrochen werden. Es wird einsichtig sein, dass die Durchführung von Messungen im wesentlichen erfordert, dass die mit höherer Drehzahl erfolgende Durchmischung unterbrochen wird und mit niedrigen Drehzahlen - und entsprechend geringen Drehmomenten - Messungen durchgeführt werden.

Da die eigentliche Messung bei einer gegebenen Drehzahl nicht lange dauern muss, und zwar selbst dann nicht, wenn über einen bestimmten Drehwinkel oder eine bestimmte Zeit hinweg gemittelt werden soll, auf Klemm-Spitzen im Drehmoment zu reagieren ist usw., die durch zwischen Mischbehälterwandung und Mischerschaufeln oder dergl. geratende grobe Körnungen usw. zurückzuführen sind, wird die Dauer der Messung im wesentlichen bestimmt sein durch jene Zeit, die erforderlich ist, um die Drehzahlen zu ändern bzw. stabile Drehzahlen zu erreichen.

Die erwähnten Klemmungen können zwischen Mischwerkzeug und Mischerwand durch grobgranulare Bestandteile und dergleichen auftreten und eine Spitze des Drehmomentes für eine gegebene Drehzahl verursachen bzw. ein Abfallen der Drehzahl- auch bis auf Null bei gegebenem Drehmoment. Diese Klemmungen lassen sich aber sehr gut in den für Drehzahl und Drehmoment indikativen Signalen erkennen. Es kann dann beispielsweise entweder das Drehmoment sehr stark hochgefahren werden oder, bevorzugt, das Mischwerkzeug ein kurzes Stück rückwärts gedreht werden, um eine Verklemmung zu lösen. Es sei erwähnt, dass sich selbst bei der Bestimmung des Losbrechmomentes Klemmspitzen gut erkennen lassen und insoweit Messungen leicht wiederholt werden können.

Es wird einzusehen sein, dass zur Erhöhung einer Drehzahl zunächst ein größeres Drehmoment erforderlich ist, um das Material in der Mischkammer auf die gewünschte Drehzahl zu beschleunigen. Erst wenn die so zunehmende Drehbewegung eine gewünschte Drehzahl ganz oder nahezu erreicht hat, bzw. sich bei gegebenem Drehmoment die Drehzahl nicht mehr (wesentlich) ändert, sollte in einer bevorzugten Variante gemessen werden.

Klassisch kann die Stabilisierung auf einer gewünschten Drehzahl durch Erreichen einer Plateauphase der Drehgeschwindigkeit bei gleichem Drehmoment festgestellt bzw. angenommen werden. Die Vorgabe eines Drehmoments ist bei der Bestimmung der sich einstellenden Drehzahlen im übrigen rheologisch bevorzugt. Es muss auch nicht eine Bestimmung der Kurve ab einem Drehmoment Null gemessen werden, sondern es reicht, bei einem Anfangswert dicht unterhalb des erwarteten Losbrech-Momentes mit der Messung zu beginnen, was die Messung zu verkürzen vermag. Das Drehmoment kann insbesondere von einem Wert größer Null, aber kleiner als das Losbrech-Moment gesteigert werden, insbesondere linear gesteigert werden, bis eine von Null verschiedene Drehzahl messbar ist.

Es ist nun einzuschätzen, dass durch die Bewegung der Mischer -Wellen bzw. MischerWerkzeuge bei der Messung nicht nur eine weitere Durchmischung erfolgen wird und diese weitere- allerdings geringe - zusätzliche Durchmischung selbst sowie die in dem frischen Beton ablaufenden Reaktionen bereits zu einer allmählichen Veränderung der Viskosität führen werden und insofern die bei Einbringung eines gegebenen Drehmomentes erzielbaren Drehzahlen nicht über sehr lange Zeiten betrachtet stabil sein werden. Bedeutsamer als chemische Reaktionen im Beton, die nur allmählich bis zum Erstarren ablaufen, ist aber, dass mit dem eingebrachten Drehmoment zunächst das Betonfluid in Bewegung gesetzt wird und sich allein dadurch das Fließverhalten und die Viskosität oftmals bereits ändert und sich ergo zunächst ein stabiler (Quasi-) Gleichgewichtszustand einstellen muss. Diese Zeit soll bei den Messungen abgewartet werden, d.h. es sollte in für die Erfindung vorteilhafter Weise unter stufenweiser Erhöhung der Drehzahlen bzw. Drehmomente gemessen werden.

Gleichwohl können Effekte wie chemische Reaktionen und zusätzliche Durchmischung für die typisch erforderlichen Messdauern von einigen wenigen Sekunden praktisch vollständig vernachlässigt werden. Dies gilt gerade dort in besonderem Maße, wo, wie bevorzugt, mit so geringen Drehgeschwindigkeiten gemessen wird, dass die Scherbelastung wenigstens 10 % unter jener liegt, die beim Mischen und/oder Pumpen der Betonmischung auftritt.

Es sei im Übrigen darauf hingewiesen, dass per se auch eine Messung in einem Oszillationsmodus möglich wäre. Während in einfacher Ausgestaltung der Erfindung eine tatsächliche Drehung der Mischerwerkzeuge in gleicher Weise wie während der eigentlichen Durchmischung erfolgt, wenn auch mit unterschiedlicher Geschwindigkeit, wird bei einer Oszillationsbewegung eine Hin- und Her-Drehbewegung nur über einen sehr kurzen Drehwinkel hinweg erfolgen.Dabei ist es per se unerheblich, wenn Mischwerkzeuge eine Vorzugsdrehrichtung, etwa aufgrund eines Anstellwinkels der Mischflächen besitzen, da gerade bei Oszillationsversuchen ein ohnehin nur geringer Drehwinkel erforderlich ist.

Per se ist diese Messung bevorzugt. Oszillations- Versuche sind präziser, dauern jedoch auch länger. Es sei erwähnt, dass Oszillationsversuche gegebenenfalls auch mit Schneckengetrieben möglich sind. Zu beachten ist allerdings, dass Spiel im Antriebszug gegebenenfalls stören kann, weil bei Oszillationsversuchen ja nur ein sehr kleiner Drehwinkel gefahren wird. Obwohl es sowohl spielarme als auch spielfreie Schneckengetriebe gibt, werden letztere einen in der Praxis oftmals zu hohen Verschleiß aufweisen, so dass gegebenenfalls etwas Spiel selbst bei Oszillationsmessungen zuzulassen ist.

Es ist möglich und bevorzugt, dass bei einem Verfahren der Erfindung vorgesehen ist, dass auf das Verhalten der leeren oder weitgehend leeren Vorrichtung dadurch kompensiert wird, dass die drehzahl- und drehmomentindikativen Signale und das Losbrechverhalten der Vorrichtung erfasst wird, zumindest bei einem einer Vorrichtung, die weniger als 0,5 % des Gesamtvolumens gefüllt wird, weniger als 1 % des Gesamtvolumens gefüllt ist, weniger als 5 % des Volumens gefüllt ist oder weniger als 10 % des Gesamtvolumens gefüllt ist.

Es möglich und bevorzugt, dass bei einem Verfahren der Erfindung das Losbrech-Verhalten dadurch kompensiert wird, dass die für Drehzahlen und Drehmomente indikativen Signale und das Losbrechen der Vorrichtung bei nur geringem Füllgrad erfasst wird. So kann es mit einer Vorrichtung erfasst werden, die weniger als 0,5 % des gesamten Volumens der Mischerkammer gefüllt ist, weniger als 1 % des Gesamtvolumens gefüllt ist, weniger als 5 % des Gesamtvolumens gefüllt ist oder weniger als 10 % des Gesamtvolumens der Mischerkammer gefüllt ist. Es sei insofern darauf hingewiesen, dass das Losbrech-Verhalten zwar abhängig davon sein kann, ob die Mischervorrichtung gefüllt ist oder nicht. So wird einzuschätzen sein, dass bei einer sehr weit gefüllten Misch-Vorrichtung nicht nur das anfänglichen Drehmoment höher sein wird, welches erforderlich ist, um eine Mischervorrichtung überhaupt in Bewegung setzen zu können, sondern dass auch die Welle stärker auf die Lager gedrückt wird und somit die Haftreibungskräfte usw. größer sind. Die Anordnung wird also bevorzugt leer sein; dabei reicht es aber ohne weiteres, wenn sie für die Bestimmung eines Losbrech-Drehmoment-Kennlinienfeldes lediglich weitgehend leer ist.

Gleichwohl ist es sinnvoll und möglich, durch Näherung für lediglich eine leere Vorrichtung das Losbrech-Verhalten zu berücksichtigen. Es sei auch darauf hingewiesen, dass das Losbrech-Verhalten einer Reihe von Einflüssen unterworfen sein wird. So ist einsichtig, dass Umgebungstemperatur und Betriebstemperaturen von Bauteilen das Losbrech-Verhalten beeinflussen können, und zwar zum einen, weil sich Viskositäten von Schmiermitteln wie Getriebeöl mit der Temperatur massiv ändern können und zum anderen auch, weil verschiedene Bauteile unterschiedliche Wärmeausdehnungen und Wärmeausdehnungskoeffizienten besitzen werden usw., was zu variierenden Belastungen nicht nur durch eine Änderung der Auflage-Kräfte führen kann, sondern auch zu - wenn auch geringen, so doch gleichwohl das Losbrech-Verhalten beeinflussenden - Deformationen usw.

Es ist möglich und bevorzugt, dass bei einem Verfahren der Erfindung vorgesehen ist, dass das Losbrechverhalten der Vorrichtung für eine Vielzahl von Betriebsbedingungen bestimmt wird, insbesondere in Abhängigkeit von der Temperatur einer oder mehrerer Komponenten, insbesondere des Getriebes oder eines Getriebeöls oder der Viskosität von Schmiermitteln, wobei bevorzugt ein Kennlinienfeld und/oder eine Nachschautabelle aufgebaut wird.

Es ist daher vorteilhaft, wenn bestimmte Parameter berücksichtigt werden und das Losbrech-Verhalten für eine Reihe unterschiedlicher Parameter, etwa in Form eines Kennlinienfeldes oder einer Nachschautabelle bestimmt wird. Zu solchen Parametern zählen insbesondere die Umgebungs-Temperatur und die Temperatur von Bauteilen oder Fluiden, die Betriebsdauer und die während des Betriebs dem Mischer zugeführte Leistung. Wenn derartige, einer Änderung unterworfene Parameter bei der Messung erfasst werden, ist das Losbrech-Verhalten genauer zu quantifizieren.

Es ist möglich und bevorzugt, dass bei einem Verfahren der Erfindung vorgesehen ist, dass die Berücksichtigung des Losbrechverhaltens der Vorrichtung erfasst wird, indem das zum Losbrechen erforderliche Drehmoment subtrahiert wird, insbesondere von den Einzelwerten bei der Erfassung einer Reihe von Messwerten mit unterschiedlicher Drehzahl und/oder unterschiedlichem Drehmomenteintrag, insbesondere durch Subtraktion von Werten aus einem Kennlinienfeld zu die jeweilige Vorrichtung charakterisierenden Größen und/oder Temperaturabhängigkeiten.

Gemäß der Erfindung wird das Losbrech-Verhalten der Vorrichtung dadurch berücksichtigt, dass das zum Losbrechen erforderliche Drehmoment - gegebenenfalls gemäß einem für die jeweiligen Betriebsbedingungen einschlägigen Wert im Kennlinienfeld oder in der Nachschautabelle - bestimmt und von dem zur Erzielung einer bestimmten Drehzahl erforderlichen Drehmoment subtrahiert wird.

Dies kann bei Erfassung einer Reihe von Messwerten insbesondere durch Subtraktion des Losbrech-Momentes von den Einzelwerten der für eine bestimmte Soll-Drehzahl erforderlichen Drehmomente erfolgen. In einem besonders bevorzugten Verfahren wird somit eine Soll-Drehzahl vorgegeben und das Drehmoment verändert, bis sich die tatsächliche Drehzahl auf die Soll-Drehzahl stabilisiert hat. Das dann erforderliche Drehmoment wird bestimmt; es wird dazu das bei den jeweiligen Bedingungen benötigte Losbrech-Drehmoment ermittelt und dieses von den Einzel-Werten subtrahiert.

Während prinzipiell einzusehen sein wird, dass ohne besondere Maßnahmen zumindest dann die gleichen Ergebnisse erhalten würden, solange das Losbrech-Verhalten nicht variiert und anstelle einer Betrachtung der Einzelwerte der Messungen durch eine einzelne Subtraktion berücksichtigt wird, ergeben sich insbesondere dann Vorteile aus der Einzelwertsubtraktion, wenn bei sehr niedrigen Drehzahlen und dementsprechend geringen Drehmomenten in einer Meßreihe zusätzlich kontrolliert wird, ob das nach Abziehen des Losbrech-Momentes zur Erzielung einer gegebenen Drehzahl bestimmte Drehmoment größer als Null ist. Wo dies nicht der Fall ist, sich also ein negatives Drehmoment zur Erzielung einer Drehzahl ergeben würde, liegt eine offensichtliche Inkonsistenz vor, die etwa durch Messwertfehler, ein zu großes angenommenes Losbrech-Moment oder dergleichen bedingt ist.

Eine solche Inkonsistenzanalyse kann dann zur Korrektur des Kennlinienfeldes und/oder momentan betrachteter Losbrech-Momente führen.

Es kann im Übrigen, und dies ist von besonderer Vorteil für die Erfindung, zu einem beliebigen Zeitpunkt während des Mischungsvorganges angenommen werden, dass eine hinreichend gründliche Durchmischung erfolgt sei.

Prinzipiell gibt es mehrere Indizien für das Erreichen einer gründlichen Durchmischung. So kann angenommen werden, dass nach einer bestimmten, ausreichend langen Zeit eine gründliche Durchmischung erzielt wurde. Als Hinweis auf das Erreichen einer gründlichen Durchmischung kann auch die Leistungsaufnahme eines Mischermotors betrachtet werden. Die Leistungsaufnahme sinkt nämlich typisch während des Mischvorganges allmählich ab und bleibt dann konstant bzw. nähert sich asymptotisch einem Endwert. Das Erreichen einer gründlichen Durchmischung kann auch durch Messung der Abhängigkeit von Drehzahlen und Drehmomenten bei niedrigen Drehzahlen insbesondere Drehzahlen unterhalb bestimmter Schwellen wie unterhalb dem 0, 5 fachen der maximalen Drehzahl, unterhalb dem 0, 5 fachen der mittleren Drehzahl über die Zeit während des Mischvorganges, usw. verifiziert werden.

Wenn nun die bei einem Verifikationsversuch bestimmten, für die Drehmomente und Drehzahlen indikativen Signale unter Berücksichtigung des Losbrech-Verhaltens der Vorrichtung z.B. dadurch darauf hindeuten, dass die Durchmischung tatsächlich gründlich genug war, dass die gewünschten Betoneigenschaften wie etwa ein bestimmtes Fließverhalten erreicht worden sind usw., kann insbesondere bei der nächsten Charge versucht werden, die angenommene Mindest-Mischzeit zu verkürzen bzw. die für das Erreichen eines Mischendes heranzuziehende Leistungsabnahme bei der nächsten Charge anders zu bewerten.

Dies kann entweder durch Verkürzung jeder einzelnen Phase des gesamten Mischvorganges erfolgen, so dass jede einzelne Phase beispielsweise um 3 oder 5 % verkürzt wird, oder aber es werden eine oder mehrere Phasen, in denen eine besonders lange und gründliche Durchmischung sinnvoll erscheint, alleine verkürzt oder stärker verkürzt als andere Phasen. So kann etwa nach Zugabe aller Komponenten mit Ausnahme einer groben Körnung ein mehrminütiger Mischvorgang gefordert werden; wenn am erwarteten Ende des Mischvorganges festgestellt wird, dass die gewünschte Betoneigenschaft wie ein bestimmtes Fließverhalten schon erreicht wurde, kann für die nächste Charge versucht werden, die Mischzeit um 10 oder 20 Sekunden der typisch wenige Minuten andauernden intensiven Mischphase zu kürzen.

Auf diese Weise ergibt sich in einem Baustellenbetrieb oder Fertigteilwerkbetrieb, wo während eines Tages viele Chargen hintereinander mit oftmals weitgehend unveränderten Zuschlagstoffen erzeugt werden, die Möglichkeit, eine kürzest mögliche Mischdauer zu bestimmen; in gleicher Weise wird, wenn festgestellt wird, dass ein Durchmischungsprozess noch nicht intensiv genug erfolgt ist, also eine Verlängerung desselben erforderlich ist, eine entsprechende Verlängerung entweder eines einzelnen Abschnittes des gesamten Vorganges oder eine gemeinsame Verlängerung mehrerer Abschnitte in gleicher oder unterschiedliche Weise vorgenommen.

Es kann so versucht werden, sich schrittweise einer optimal kurzen Durchmischungsdauer anzunähern, wobei durch Betrachtung sich als zu lang oder zu kurz erwiesen habender Mischdauern, gegebenenfalls unter Berücksichtigung, dass sich im Laufe eines Tages durch Temperaturänderungen usw. Änderungen ergeben können, aufgrund von Schwankungen der Eigenschaften verwendete natürliche Materialien usw. Änderungen der Mindestmischdauer auch auftreten können usw., die Gesamteffizienz der Anlage optimiert wird.

Schutz wird auch beansprucht für eine Beton-Mischvorrichtung mit einem Mittel zur Erfassung von für Drehzahlen und Drehmomente indikativen Signalen und einem Mittel zur Bestimmung einer Betoneigenschaft unter Berücksichtigung eines Losbrech-Verhaltens.

Es wird verständlich sein, dass die Mittel zur Erfassung von Signalen, die für die Drehzahlen und Drehmomente der Mischvorrichtung indikativ sind, Drehzahlsensoren wie Drehzahlmessgeber - beispielsweise an zumindest einer von Antriebswelle, Mischerwelle und Getriebewelle - umfassen können und dass die Mittel zur Erfassung von für Drehmomente indikativen Signalen insbesondere zur Erfassung eines Antriebsstromes und/oder einer Antriebsleistung eines zum Antrieb einer Mischerwelle verwendeten Elektro-Motors ausgebildet sein können. Es sei diesbezüglich erwähnt, dass verschiedene Arten von (Elektro-) Motoren für die Erfindung verwendet werden können. In vielen Fällen werden Asynchron-Motoren bevorzugt sein. Zu beachten ist bei der Motorwahl dazu einerseits der Wirkungsgrad, der etwa für Asynchron-Motoren und die zugehörigen Umrichter sehr hoch sein kann, wenn sie korrekt an eine jeweilige Antriebsaufgabe angepasst sind, wobei aber der Wirkungsgrad bei Asynchron-Motoren zurückgeht, wenn der Motor nur unter Teillast betrieben wird, was zumindest für einen Teil der Mischphase der Fall sein wird. Zudem muss beachtet werden, dass der Antrieb zur Erzielung eines hinreichend großen Losbrech-Momentes ausgelegt sein muss, was eine entsprechend große Dimensionierung sowohl des Umrichters als auch des Motors erfordert.

Was den Motorantrieb angeht, so sei explizit auf die Möglichkeit hingewiesen, für die vorliegende Erfindung einen Motor mit DTC (direct torque control; direkter Drehmomentsteuerung) zu verwenden. Bei diesem ist der zur Speisung von Antriebsstrom an den Elektromotor vorgesehene Umrichter dazu ausgebildet, den Antrieb auf optimierte Weise zu steuern.

Es wird einzuschätzen sein, dass gerade für die Mischung von Hochleistungsbeton hohe Antriebsleistungen im Bereich von mehreren 10kW, z.B. von 20kW bis 100kW für Mischvolumina (Chargengrößen) um 200 Liter bis 1000 Liter vorteilhaft sind. Dabei ist zu beachten, dass ein Anlaufdrehmoment hinreichend hoch sein muss und neben dem eigentlichen Antriebsmotor auch die zugehörigen Komponenten wie der Frequenz-Umrichter und die weiteren, im Antriebsstrang vorhanden Komponenten dafür ausgelegt sein sollen, zumindest kurzfristig Leistungsspitzen bereitstellen zu können.

Es kann entweder ein aktueller Sollwert für Strom bzw. Leistung als ein für Drehmomente indikatives Signal herangezogen werden, insbesondere insofern, als ein derartiger Sollwert von einer Steuerung vorgegeben wird, oder aber es werden die aktuellen, für Drehmomente indikativen Signale bestimmt, d.h. die aktuellen Leistungen bzw. Ströme. Die Mittel zur Berücksichtigung des Losbrech-Verhaltens der Vorrichtung bei der Bestimmung der Betoneigenschaft im Ansprechen auf die für die Drehzahlen bzw. Drehmomente indikativen Signale können auch durch eine digital arbeitende Stufe wie einen Prozess-Rechner oder dergl. gebildet sein, in welchen digitale Signale eingespeist werden, die für die Drehzahlen bzw. Drehmomente indikativ sind; im Prozessrechner erfolgt dann eine Verknüpfung derartiger Signale mit Operanden, welche repräsentativ für ein zu betrachtendes Losbrech-Verhalten der Vorrichtung sind.

Mit einer solchen Anordnung ist es möglich, die Mischvorrichtung zu steuern und/oder eine manuelle Steuerung zu unterstützen, z.B. indem Signale ausgegeben werden, die die Fertigstellung einer Mischung anzeigen, die eine Entleerung der Vorrichtung in einen bereitstehenden Transportbehälter zum Transport an einen Verwendungsort auslösen und/oder anfordern, und/oder die darauf hinweisen, dass eine Fortsetzung der Durchmischung, evtl. unter Zusatz von Zusatzmitteln erforderlich ist, die insbesondere bevorzugt auch automatisch bestimmt werden können, im Ansprechen auf Art und/oder Größe einer Abweichung zwischen Ist- und Sollverhalten des Betons und so weiter.

Es sei im übrigen erwähnt, dass eine solche Steuerung auch, insbesondere selbsttätig, ein Mischprogramm abfahren kann, bei welchem zu bestimmten Zeitpunkten verschiedene Beton-Bildner in die Mischvorrichtung gegeben werden, also z.B. Wasser, Zement, Zuschlagstoffe und so weiter, und zwar gegebenenfalls auch sukzessive. Zugleich kann ein bestimmtes Mischwellengeschwindigkeits-Programm abgefahren werden und/oder die Zusatzstoffe und/oder Zusatzmittel bestimmt werden, die zur Erzielung bestimmter gewünschter Betoncharakteristika noch zugesetzt und eingemischt werden müssen.

Es kann also nach dem Vorstehenden ein frisch hergestelltes Betongemisch beispielsweise nach Durchführung einer Mischphase durch das rheologische Verhalten im Bereich niedriger Drehzahlen bzw. hinreichend niedriger Drehmomente charakterisiert werden. In einem praktischen Beispiel kann dazu für einen typischen selbstverdichtenden Beton etwa 60 Sekunden in einem Doppelwellenmischer gemischt werden. Der während dieser Zeit dem Mischer-Motor zuzuführende Strom sinkt kontinuierlich ab, wobei eine praktisch asymptotische Näherung an einen Grenzwert erfolgt und eine hinreichende Annäherung an den Grenzwert anzeigt, dass der eigentliche Durchmischungsprozess abgeschlossen ist.

Nach dem eigentlichen Durchmischungsprozess, d.h. dann, wenn sich die Mischung durch reines Vermengen nicht weiter verändert, wird der frisch angesetzte Beton charakterisiert, um zu prüfen, ob geforderte Eigenschaften erreicht worden sind. Ist dies nicht der Fall, werden Zusätze je nach Abweichung automatisch bestimmt bzw. durch einen Anlagenbetreiber eine Zugabe von Zusätzen veranlasst und ergo die Mischung durch Zusätze verändert. Für den Test, ob die Eigenschaften erreicht wurden, wird das Fließverhalten bei selbstverdichtendem Beton bei sehr niedrigen Drehmomenten knapp oberhalb des Losbrech-Momentes bestimmt, d.h. jenes Drehmomentes, das auf die Mischerwelle anzulegen ist, damit diese die anfängliche, in Lagern und dem zu messenden Material auftretende Reibung überwindet und sich in Bewegung setzt. Bestimmt werden kann z.B. die Fließgrenze und die Viskosität.

Die Erfindung wird im Folgenden nur beispielsweise anhand der Zeichnung beschrieben. In dieser ist dargestellt durch
- Figur 1a: Scherraten und Scherspannungen, wie sie während rheologischer Messungen und während der Herstellung bzw. des Pumpens von Beton auftreten;
- Fig. 1b: ein vergrößerten Ausschnitt aus Fig. 1a;
- Figur 2: eine Anordnung zur Ausführung des Verfahrens der vorliegenden Erfindung;

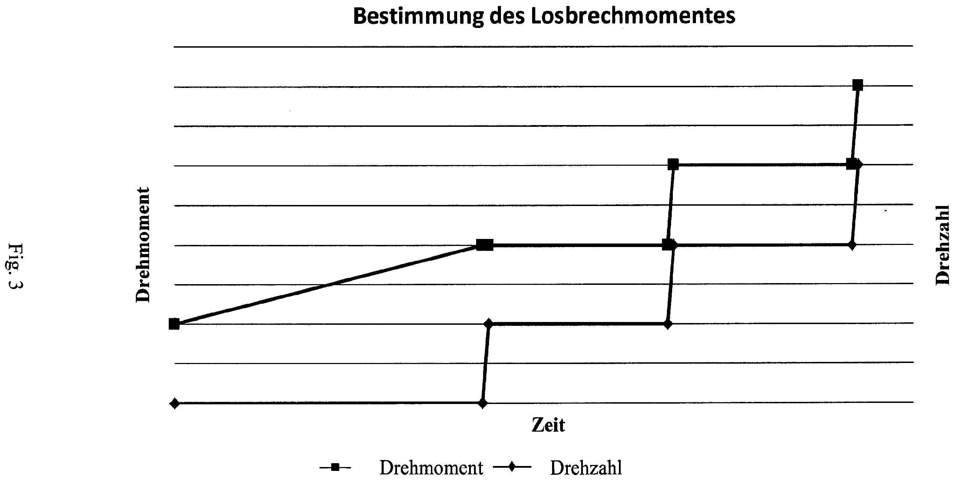

- Figur 3: der Verlauf von Drehmoment und Drehzahl während der Ausführung einer bevorzugten Variante eines erfindungsgemäßen Verfahrens.

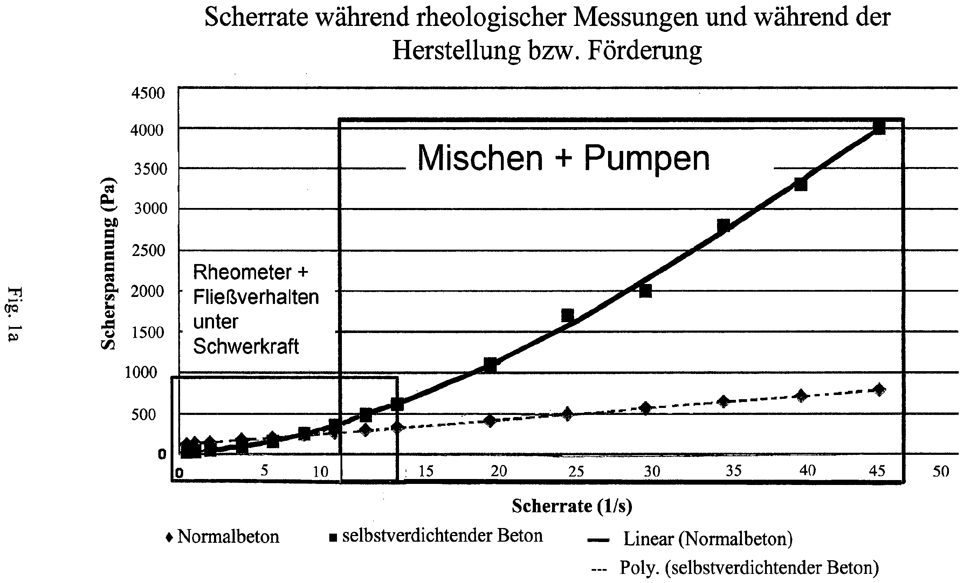

Figur 1a zeigt zunächst für Normalbeton (Rauten, untere Kurve) sowie für selbstverdichtenden Beton (Vierecke, obere Kurve) Messwerte der Scherspannung gegen die Scherrate. Aufgetragen sind dabei Messwerte und Ausgleichskurven durch diese, wobei für den Normalbeton ein linearer Zusammenhang angenommen wurde, während für die Messwerte, welche zu dem selbstverdichtenden Beton gehören, die Kurve mit einem Polynom genähert wurde.

Weiter sind in der Figur 1a Fenster eingezeichnet, die angeben, in welchem Bereich von Scherraten und Scherspannungen typisch Misch- bzw. Pumpprozesse einerseits und andererseits Mess-Prozesse in Rheometern sowie Fließvorgänge unter Schwerkraft liegen werden. Es ist ersichtlich, dass typische Misch- bzw. Pump-Prozesse bei deutlich höheren Scherraten und entsprechend höheren Scherspannungen ablaufen, als sie beim Fließen von frischem Beton unter Schwerkraft auftreten, auch wenn ein geringer Überlapp der Fenster auftritt.

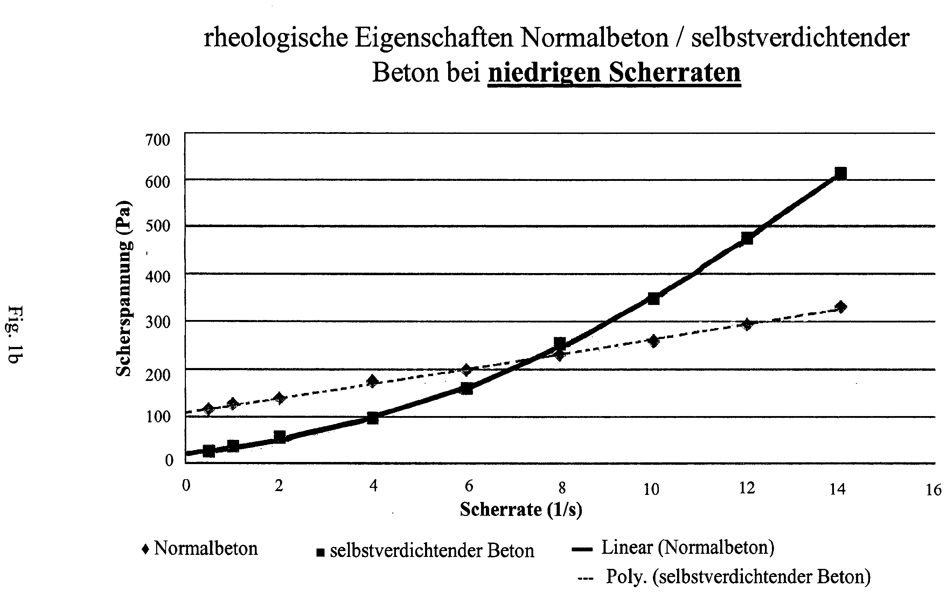

Überdies ist erkennbar, dass die bei selbstverdichtendem Beton insbesondere bei höheren Scherraten zu beobachtenden Scherspannungen deutlich höher sind als jene von Normalbeton. Weiter ist zu erkennen, dass bei selbstverdichtendem Beton kein linearer Zusammenhang zwischen Scherspannung und Scherrate besteht. Es sei erwähnt, dass die in Figur 1 gezeigten Kurven ohne weiteres mit Mitteln aus dem Stand der Technik verifizierbar sind.

Figur 1a zeigt, dass für eine präzise Charakterisierung insbesondere von selbstverdichtendem Beton nicht aus den bei hohen Scherraten ermittelten Werten extrapoliert werden sollte. Dies lässt auch Figur 1 b erkennen, wo ein Ausschnitt der Kurven noch einmal vergrößert dargestellt ist. Es wird insbesondere einzusehen sein, dass die extrapolierten Kurven nicht exakt durch den Ursprung verlaufen.

Vor diesem Hintergrund schlägt die vorliegende Erfindung nun vor, eine Vorrichtung zum Mischen von Beton zu verbessern.

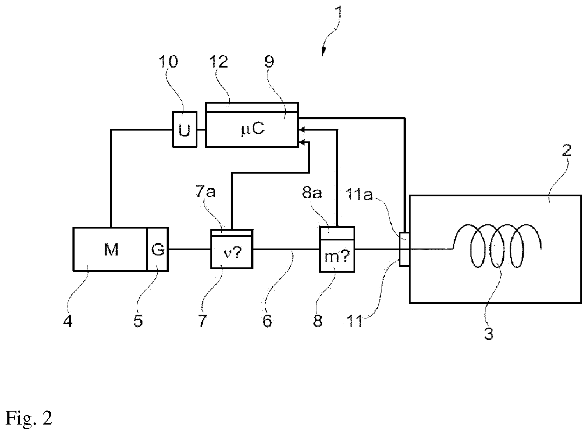

Nach Figur 2 umfasst eine allgemein mit 1 bezeichnete Mischvorrichtung für Beton eine Mischkammer 2, in welcher eine Mischerwelle 3, vorliegend dargestellt als Einzelwelle 3, vorgesehen ist, die von einem Motor 4 über ein Getriebe 5 angetrieben wird.

Bei der Antriebswelle 6 zwischen Getriebe 5 und Mischerwelle 3 ist einerseits ein Sensor 7 zur Bestimmung von Drehzahlen bzw. von drehzahlindikativen Signalen und ein Sensor 8 zur Bestimmung eines Antriebsdrehmomentes, das heißt von drehmomentindikativen Signalen vorgesehen.

Die Sensorsignale aus dem Sensor zur Bestimmung der drehzahlindikativen Signale 7 werden in einer Signalkonditionierungsstufe 7a konditioniert und an eine Prozessrechnereinheit 9 gespeist.

Die Drehmomentindikativsignale aus dem Sensor 8 werden in einer Signalkonditionierungsstufe 8 a konditioniert und ebenfalls an die Prozessrechnereinheit 9 gespeist, so dass die Prozessrechnereinheit 9 sowohl drehzahlindikative Signale empfängt als auch drehmomentindikative Signale empfängt. Die Prozessrechnereinheit 9 gibt Steuerbefehle an einen Umrichter 10 aus, aus welchem der Motor 4 mit Antriebsleistung versorgt wird.

Es sei erwähnt, dass die Sensoren 7 und 8 nicht zwingend im Antriebszug vorgesehen sein müssen, sondern stattdessen auch auf andere Weise drehmomentindikative Signale und drehzahlindikative Signale erzeugt werden können, beispielsweise durch Bestimmung entsprechender Kenngrößen am Umrichter 10 bzw. am Motor 4 und es ist möglich, dort, wo dedizierte Sensoren vorgesehen sein sollen, diese nicht zwischen Getriebe 5 und Mischerwelle 3, sondern beispielsweise auch zwischen Motor 4 und Getriebe 5 vorzusehen.

Es sei weiter erwähnt, dass Leistungsversorgungs-Leitungen usw. nicht eingezeichnet sind.

Die Mischerkammer 2 hat im vorliegenden Ausführungsbeispiel eine Größe von 500 Liter Chargenvolumen.

Der Motor 4 ist im vorliegenden Fall ein Asynchronmotor und der Umrichter 10 eine DTCfähige Umrichtereinheit.

Das Getriebe 5 ist im vorliegenden Fall ein Getriebe, das mit Schmiermitteln geschmiert wird. Die Viskosität der Schmiermittel ändert sich mit deren Temperatur und die Schmiermittel-Temperatur ändert sich wiederum mit sowohl der Umgebungstemperatur als auch mit der Betrieb stemperatur.

Um dies zu berücksichtigen, wird nun die Temperatur am Getriebe (sowie an weiteren kritischen Stellen der Vorrichtung, vorliegend dargestellt beispielhaft an den Mischerwellen-Lagern) mit Temperatursensoren 11 gemessen und die entsprechenden Signale in einer Signalkonditionierungsstufe 11a konditioniert und wiederum an die Prozessrechnereinheit 9 gespeist.

In der Prozessrechnereinheit 9 ist ein Speicher 12 mit einer Nachschautabelle vorhanden, in welcher für verschiedene Betriebstemperaturen, wie sie mit den Temperatursensoren 11 erfasst werden, die Losbrech-Momente der Vorrichtung für den Fall abgelegt sind, dass die Vorrichtung nicht mehr als 1 % gefüllt ist, d.h. zumindest weitgehend leer läuft. Diese Leer-Losbrech-Kennwerte können, wie noch beschrieben werden wird, von der Prozessrechnereinheit verrechnet werden.

Die Antriebsleistung des Motors 4 ist so hoch, dass eine Charge bei Herstellung von Ultrahochleistungsbeton binnen ca. 2 Minuten gründlich durchmischt werden kann.

Es sei erwähnt, dass der Vorrichtung weitere Einheiten wie Doseure zugeordnet sind, aus denen kontrollierte Materialmengen zu gewünschten Zeiten in die Mischkammer 2 eingebracht werden können, und zwar unter automatischer Steuerung durch die Prozessrechnereinheit 9. Weiter sei darauf hingewiesen, dass die Mischkammer 2 automatisch unter der Steuerung der Prozessrechnereinheit 9 in einen Transportbehälter entleert werden kann.

Um mit der Vorrichtung 1 nun ein Verfahren zur Herstellung von Beton durchzuführen, wobei für Drehzahl und Drehmoment indikative Signale an der Vorrichtung 1 erfasst werden und zumindest eine Betoneigenschaft im Ansprechen darauf durch die Prozessrechnereinheit 9 bestimmt wird, soll das Losbrech-Verhalten der Vorrichtung 1 bestimmt werden, d.h. berücksichtigt werden, bei Eintragen welchen Drehmomentes sich die Vorrichtung zu drehen beginnt.

Dies geschieht wie folgt:
Zunächst wird eine Charge (Ultra-) Hochleistungsbeton nach einem vorgegebenen Programm erzeugt, wobei die einzelnen Komponenten des Betons wie erforderlich in die Mischkammer gegeben werden und die Mischerwelle 3 vom Motor 4 über die Getriebewelle 5 durch Erregen des Umrichters angetrieben wird.

Dies setzt sich fort, bis sich die Leistungsaufnahme des Motors 4 zur Erzielung einer gegebenen Drehzahl der Mischerwelle 3 nach Zugabe aller Beton-Ausgangsstoffe hinreichend nah einem asymptotischen Grenzwert genähert hat, was vom Prozessrechner 9 durch Überwachung der Drehzahl unter Verwendung des Sensors 7 bestimmt werden kann.

Sobald dies der Fall ist, wird der anfängliche Mischprozess beendet und ein Mess-Zyklus eingeleitet. Bei diesem Mess-Zyklus wird, ausgehend von einer ruhenden Mischerwelle so lange ein linear mit der Zeit ansteigendes Drehmoment auf die Mischerwelle 3 gegeben, bis die Mischerwelle sich zu drehen beginnt. Es wird einzuschätzen sein, dass zunächst, das heißt bei geringen Drehmomenten, keine Drehung erfolgen wird und die Mischerwelle erst dann, wenn ein hinreichend hohes Drehmoment anliegt, anzulaufen beginnt.

Zu diesem Zeitpunkt bzw. bei diesem Drehmoment tritt also ein Losbrechen auf.

Dann wird zunächst, d.h. nach dem Losbrechen der Vorrichtung nach Steigerung des Drehmoments und bis zum Losbrechen die Drehzahl für eine bestimmte Zeit konstant gehalten, um sicherzustellen, dass eine Messung des bei dieser Drehzahl erforderlichen Drehmoments nicht durch Effekte wie Beschleunigen der Drehung beeinflusst ist.

Es wird nun einsichtig sein, dass bei der gefüllten Mischkammer ein größeres Drehmoment erforderlich ist als bei leerer Mischkammer. Auch wird einsichtig sein, dass bei einer gefüllten Mischkammer 2 ein Drehmoment, bei welchem Losbrechen auftritt, von verschiedenen Größen abhängen wird. So wird einsichtig sein, dass bei einer gefüllten Mischkammer ein größeres Drehmoment erforderlich ist als bei einer leeren Mischkammer. Zudem wird - nur beispielhaft genannt- die Viskosität von Schmierölen in den Lagern relevant sein; eine solche Viskosität wird sich mit der Temperatur ändern. Demnach wird bei einer gefüllten Mischkammer 2 das Drehmoment, das für Losbrechen erforderlich ist, von verschiedenen Größen abhängen, wie z.B. der Viskosität von Schmierölen in den Lagern und Getrieben; da sich eine solche Viskosität wiederum mit der Temperatur ändern wird, wird während der Drehzahl-bzw. Drehmoment-Haltephase an den einschlägigen Stellen mit den Temperatursensoren 11 die Temperatur erfasst und an die Prozessrechnereinheit gespeist. Damit liegt zu den drehmomentindikativen und drehzahlindikativen Signalen auch die Temperatur vor, bei welcher die drehmomentindikativen und drehzahlindikativen Signalen erfasst wurden.

Nach einer bestimmten Haltephase, während welcher die Drehzahl und das Drehmoment und ergo die drehzahlindikativen und drehmomentindikativen Signale, die an die Prozessrechnereinheit 9 gespeist werden, praktisch konstant sind, d.h. sich um nicht mehr als einen zulässigen Prozentsatz wie beispielsweise 3 % geändert haben, wird sprungartig das Drehmoment erhöht und wieder abgewartet, bis sich die Drehzahl wieder stabilisiert hat.

Es wird wiederum eine Haltephase abgewartet, während welcher Drehmoment- und drehzahlindikative Signale ergo wieder praktisch konstant sind und von der Prozessrechnereinheit erfasst werden. Wiederum werden auch die Temperaturen durch Sensoren 11 bestimmt.

Dies setzt sich im Bereich niedriger Drehzahlen, wie sie für das Fließverhalten typisch sind, mit wenigstens 5, vorliegend 10 Messwerten fort.

Danach wird in größeren Schritten ein Drehmoment eingetragen, um Messungen unter Bedingungen durchführen zu können, wie sie für das Mischen der Betonmischung typisch sind. Es sei hier erwähnt, dass die bei Mischen der Betonmischung erreichbaren Scherraten typisch deutlich unterhalb jener Scherraten liegen werden, die beim Pumpen des Betons ausgeübt werden. Nun kann die Charakterisierung des Betonverhaltens während des Pumpens sinnvoll sein, um sicherzustellen, dass an einer Baustelle eine gegebene Betoncharge ein ordnungsgemäßes Verhalten aufweisen wird. Ungeachtet dessen, dass mit typischen Mischern die Scherraten beim Pumpen nicht erreicht werden, ist eine sinnvolle Charakterisierung insoweit möglich, als die in einem Mischer noch erzielbaren Scherraten auf jene Scherraten extrapolierbar sind, wie sie der Beton beim Pumpen erfährt. Gerade dort, wo ein Pumpverhalten oder ein anderes Verhalten mit evaluiert werden soll, das bei höheren Scherraten Bedeutung gewinnt, ist es demnach sinnvoll, (zusätzlich) Messwerte auch bei höheren Drehmomenten bzw. Scherraten zu erfassen. Auch wenn sich dort das Losbrechverhalten weniger deutlich auswirkt, kann es doch vorteilhaft sein, das Losbrechverhalten zu berücksichtigen. Ausschlaggebend dafür ist zunächst, dass sonst durch Interpolation zwischen hohen und niedrigen Scherraten die bei kleineren Scherraten interpolierten Werte doch verfälscht werden. Schon daher ist es vorteilhaft, wenn auch nicht zwingend, auch für größere Scherraten die das Losbrechverhalten beeinflussenden Größen zu bestimmen und das Losbrechverhalten entsprechend zu berücksichtigen. Zudem wird einsichtig sein, dass durch die erfindungsgemäße Berücksichtigung des Losbrechverhaltens selbst bei großen Scherraten bzw. Drehmomenten nicht nur indirekt, sondern per se Vorteile erhalten werden, weil eine höhere Präzision erreicht wird.

Nach Abschluss dieser Messphase liegen somit zunächst eine Reihe von Messwerten vor, bei denen Drehmomente und Drehzahlen ermittelt wurden und zu denen die jeweiligen Betriebsbedingungen der Vorrichtung, welche das Losbrechen Verhalten beeinflussen können, bekannt sind.

Es kann nun für jeden der bestimmten Messwerte ermittelt werden, wie groß zum Erreichen einer bestimmten Drehzahl das Drehmoment korrigiert um jenes Drehmoment sein muss, das bei den - vorliegend mit den Temperatursensoren 11 ermittelten- Betriebsbedingungen - bereits bei einer leeren Vorrichtung 1 für ein Losbrechen erforderlich wäre; es kann dann durch die entsprechenden, auf das (Leer-)-Losbrech-Moment korrigierten Messwerte eine Ausgleichskurve gelegt werden, wobei zugleich geprüft wird, ob bestimmte Messwerte durch Ausreißer verfälscht sind, um entweder die Messung wiederholen zu können und oder derartige Messwerte verwerfen zu können. Es sei erwähnt, dass nicht unmittelbar eine Ausgleichskurve durch Drehzahlen und (korrigierte) Drehmomente gelegt werden muss, sondern dass andere Zusammenhänge hergestellt werden können und im übrigen aus den Messwerten wie erwünscht und bekannt das Fließverhalten des Betons charakterisierende Größen ermittelt werden können.

Sofern sich gemäß einer solchen Auswertung Abweichungen von einer Soll-Charakterisierung ergeben, können erforderliche Zusatzstoffe automatisch bestimmt werden, in die Mischkammer eingebracht werden und der eigentliche Mischprozess zur Einmischung derselben wieder aufgenommen werden; nach einer bestimmten Zeit oder nach Erreichen eines anderen Kriteriums kann dann eine neuerliche Messung der rheologischen Eigenschaften wie vorstehend beschrieben durchgeführt werden.

Es wird damit eine sehr genaue Charakterisierung des Fließverhaltens von selbstverdichtenden Beton möglich und die Herstellung eines Hochleistungsbetons mit hervorragend reproduzierbaren Eigenschaften.

## Patentansprüche

1. Verfahren zur Herstellung von Beton mittels einer Mischvorrichtung,
an der
drehzahl- und drehmomentindikative Signale erfasst werden
und eine Betoneigenschaft im Ansprechen darauf bestimmt wird,
**dadurch gekennzeichnet, dass**
bei der Bestimmung der Betoneigenschaft das Losbrechverhalten der Vorrichtung berücksichtigt wird,
indem das zum Losbrechen erforderliche Drehmoment ermittelt wird und von dem zur Erzielung einer bestimmten Drehzahl erforderlichen Drehmoment abgezogen wird.

2. Verfahren zur Herstellung von Hochleistungsbeton und/oder selbstverdichtendem Beton nach dem vorgehenden Anspruch, bevorzugt mit einem Volumen je Charge aus der Gruppe von größer 100 Liter, größer 200 Liter, größer 500 Liter, größer 1000 Liter und/oder bevorzugt in einer festen oder transportablen Mischvorrichtung.

3. Verfahren nach einem der vorhergehenden Ansprüche, **dadurch gekennzeichnet, dass** als Mischvorrichtung einer aus der Gruppe Mischer mit nur einem Mischwerkzeug, Mischer mit einer einzelnen Gruppe von im Verbund beweglichen Mischwerkzeugen, Ringtrogmischer, Tellennischer, Flanetenmischer, Einzel- oder Doppelwellenmischer verwendet wird, bevorzugt mit einem Getriebe aus der Gruppe Planetengetriebe, Schneckengetriebe und/oder Riemenscheibe zwischen Antriebswelle(n) und einem oder mehreren Antriebsmotoren.

4. Verfahren nach einem der vorhergehenden Ansprüche, **dadurch gekennzeichnet, dass** zur Messung drehzahl- und drehniiomentindikativer Signale
ein Drelizahimessgeber an einem von
Mischerwelle,
Getriebewelle und
Antriebsmotor
vorgesehen ist,
und/oder drehmomentindikative Signale
vor
oder nach einem Getriebe
erfasst werden, bevorzugt durch Erfassung
einer eingespeisten elektrischen Antriebsleistung
und/oder Umrichtefrequenz für den Mischerwellen-Antrieb.

5. Verfahren nach einem der vorhergehenden Ansprüche, **dadurch gekennzeichnet, dass**
die drehzahl- und drehmomentindikativen Signale
an zumindest einem von
Getriebe,
Motor und
Mischerwelle(n)
erfasst werden
und/oder
die Erfassung die
Einspeisung
der digitalisierten und konditionierten drehzahl- und
drehmomentindikativen Signale
in eine Prozessrechnereinheit amfasst.

6. Verfahren nach einem der vorhergehenden Ansprüche, **dadurch gekennzeichnet, dass** als Betoneigenschaft zumindest das Fließverhalten des Betons bestimmt wird.

7. Verfahren nach einem der vorgehenden Ansprüche, **dadurch gekennzeichnet, dass** die Erfassung der drehzahl- und drehmomentindikativen Signale die Messung einer Reihe von wenigstens 2, bevorzugt wenigstens 3, besonders bevorzugt wenigstens 5 Messwerten umfasst, die erfasst werden bei einem aus der Gruppe Drehzahlen bis zum 0,25fachen der maximalen Drehzahl während des Mischungsvorganges; Drehzahlen bis zum 0,25fachen der mittleren Drehzahl über die Zeit während des Mischungsvorganges; und dass aus der Reihe von wenigstens 2, bzw. wenigstens 3, bzw. wenigstens 5 Messwerten eine Interpolation gegen die Drehzahl N = 0 erfolgt; und/oder dass die Erfassung der drehzahl- und drehmomentindikativen Signale an der Vorrichtung intermittierend zum Mischvorgang erfolgt, bevorzugt bei einer
Scherbelastung, die mindestens 10 % unter jener liegt, wie sie beim Mischen der Betonmischung maximal, bevorzugt im Mittel auftritt, besonders bevorzugt bei einer Scherbelastung von maximal 25 % jener, wie sie beim Mischen auftritt.

8. Verfahren nach einem der vorhergehenden Ansprüche, **dadurch gekennzeichnet, dass** zur Bestimmung der drehzahl- und drehmomentindikativen Signale an der Vorrichtung das Drehmoment stufenweise erhöht wird und dann abgewartet wird, bis die Drehzahl sich stabilisiert hat und eine Plateauphase erreicht ist.

9. Verfahren nach einem der vorhergehenden Ansprüche, **dadurch gekennzeichnet, dass** das Losbrechverhalten dadurch kompensiert wird, dass das Losbrechverhalten mit einer leeren oder weitgehend leeren Vorrichtung erfasst wird und die drehzahl- und drehmomentindikativen Signale bei gefüllter Vorrichtung darauf korrigiert werden, wobei bevorzugt die leere oder weitgehend leere Vorrichtung bei Bestimmung des Leer-Losbrechverhaltens weniger als 1 0 % ihres Mischkammervolumens gefüllt ist, weiter bevorzugt weniger als 5 % des Volumens gefüllt ist, weiter bevorzugt weniger als 1 % des Volumens gefüllt ist und besonders bevorzugt weniger als 0,5 % des Volumens gefüllt ist.

10. Verfahren nach einem der vorhergehenden Ansprüche, worin das Losbrechverhalten der Vorrichtung für eine Vielzahl von Betriebsbedingungen bestimmt wird, insbesondere in Abhängigkeit von der Temperatur einer oder mehrerer Komponenten, insbesondere des Getriebes und/ oder eines Getriebeöls und/ oder der Viskosität von Schmiermitteln, wobei bevorzugt ein Kennlinienfeld und/oder eine Nachschautabelle aufgebaut wird und eine Kompensation auf das Losbrechmoment unter Berücksichtigung der Betriebsbedingungen erfolgt.

11. Verfahren nach einem der vorhergehenden. Ansprüche, **dadurch gekennzeichnet, dass** die Berücksichtigung des Losbrechverhaltens der Vorrichtung erfolgt, indem das zum Losbrechen erforderliche Drehmoment von einzelnen einer Reihe von Messwerten subtrahiert wird, die mit unterschiedlichen Drehzahlen und/oder unterschiedlichem Drehmomenteintrag aufgenommen werden, insbesondere durch Subtraktion einer aus einem Kennlinienfeld oder einer Vorrichtungscharakterisierung hergeleiteten Größe und/oder Temperaturabhängigkeit.

12. Mischvorrichtung (1)
zur Ausführung eines Verfahrens nach einem der vorhergehenden Ansprüche mit
einem Mittel (7,8) zur Erfassung von für Drehzahlen und Drehmomente indikativen Signalen
und
einem Mittel zur Bestimmung zumindest einer Betoneigenschaft im Ansprechen darauf,
**dadurch gekennzeichnet, dass**
das Mittel (9) zur Bestimmung zumindest einer Betoneigenschaft im Ansprechen auf für Drehzahlen und Drehmomente indikative Signale
als Mittel zur Bestimmung zumindest einer Betoneigenschaft im Ansprechen auf für Drehzahlen und Drehmomente indikative Signale unter Berücksichtigung des Losbrechverhaltens dazu ausgebildet ist, das zum Losbrechen erforderliche Drehmoment zu ermitteln und von dem zur Erzielung einer bestimmten Drehzahl erforderlichen Drehmoment abzuziehen,
um so eine Bestimmung der Betoneigenschaft
im Ansprechen auf
sowohl die für Drehzahlen und Drehmomente indikativen Signale
als auch das Losbrechverhalten
vornehmen zu können.

13. Mischvorrichtung (1) nach dem vorhergehenden Anspruch, **dadurch gekennzeichnet, dass** das Mittel (9) zur Bestimmung zumindest einer Betoneigenschaft im Ansprechen auf für Drehzahlen und Drehmomente indikative Signale als Prozessrechner ausgebildet ist, der für Einspeisung der digitalisierten und konditionierten drehzahl- und drehmomentindikativen Signale ausgebildet ist.

## Claims

1. Method for producing concrete by means of a mixing device, on which
speed- and torque-indicative signals are recorded and a property of the concrete is determined in response thereto,
**characterized in that**
the breakaway behavior of the device is taken into account in the determination of the property of the concrete
by the torque that is required for breaking away being ascertained and subtracted from the torque required to achieve a specific speed.

2. Method for producing high-performance concrete and/or self-compacting concrete according to the preceding claim, preferably with a volume per batch from the group comprising greater than 100 litres,
greater than 200 litres, greater than 500 litres, greater than 1000 litres and/or preferably in a fixed or transportable mixing device.

3. Method according to either of the preceding claims, **characterized in that** the mixing device used is one from the group comprising mixers with only one mixing tool, mixers with a single group of mixing tools movable together, a ring trough mixer, a pan mixer, a planetary mixer, a single- or double-shaft mixer, preferably with a gear mechanism from the group comprising a planetary gear mechanism, a worm gear mechanism and/or a belt pulley between drive shaft(s) and one or more drive motors.

4. Method according to one of the preceding claims, **characterized in that**, for measuring speed- and torque-indicative signals, a tachometer is provided on either one of the following
the mixer shaft,
the gear shaft and
the drive motor,
and/or torque-indicative signals are recorded upstream
or downstream of a gear mechanism,
preferably by recording
a fed-in electrical drive power
and/or converter frequency for the mixer-shaft drive.

5. Method according to one of the preceding claims, **characterized in that** the speed- and torque-indicative signals are recorded
on at least one of the following
the gear mechanism,
the motor and
the mixer shaft(s)
and/or
the recording comprising
the feeding
of the digitized and conditioned speed- and torque-indicative signals
into a process computer unit.

6. Method according to one of the preceding claims, **characterized in that** at least the flow behaviour of the concrete is determined as the property of the concrete.

7. Method according to one of the preceding claims, **characterized in that** the recording of the speed-and torque-indicative signals comprises the measuring of a series of at least 2, preferably at least 3, particularly preferably at least 5, measured values, which are recorded at a speed from the group speeds up to at 0.25 times the maximum speed during the mixing operation;
speeds up to 0.25x the average speed over the time during the mixing operation; and **in that** from the series of at least 2, or at least 3, or at least 5,
measured values, an interpolation is performed towards the speed N = 0; and/or **in that** the recording of the speed- and torque-indicative signals takes place on the device intermittently with respect to the mixing operation, preferably under a shear loading that lies at least 10% below that which occurs as a maximum, preferably on average, during the mixing of the concrete mixture, particularly preferably under a shear loading of a maximum of 25% of that which occurs during the mixing.

8. Method according to one of the preceding claims, **characterized in that**, for determining the speed-and torque-indicative signals on the device, the torque is increased in stages and then left for the time it takes until the speed has stabilized and a plateau phase is reached.

9. Method according to one of the preceding claims, **characterized in that** the breakaway behaviour is compensated by the breakaway behaviour with an empty or largely empty device being recorded and the speed- and torque-indicative signals with the filled device being corrected on that basis, wherein preferably in the determination of the empty breakaway behaviour the empty or largely empty device is filled less than 10% of its mixing chamber volume, more preferably is filled less than 5% of the volume, more preferably is filled less than 1% of the volume and particularly preferably is filled less than 0.5% of the volume.

10. Method according to one of the preceding claims, in which the breakaway behaviour of the device is determined for a large number of operating conditions, in particular in dependence on the temperature of one or more components, in particular of the gear mechanism and/or a gear oil and/or the viscosity of lubricants, wherein preferably a characteristic map and/or a lookup table is set up and compensation for the breakaway torque takes place while taking the operating conditions into account.

11. Method according to one of the preceding claims, **characterized in that** the breakaway behaviour of the device is taken into account by the torque required for breaking away being subtracted from individual values of a series of measured values that are recorded at different speeds and/or with different torque input, in particular by subtraction of a variable derived from a characteristic map or a device characterization and/or temperature dependence.

12. Mixing device (1)
for performing a method according to one of the preceding claims, with
a means (7, 8) for recording
signals
indicative of speeds and torques
and
means for determining at least one property of the concrete in response thereto,
**characterized in that**
the means (9) for determining at least one property of the concrete in response to signals indicative of speeds and torques
is designed as a means for determining at least one property of the concrete in response to signals indicative of speeds and torques while taking into account the breakaway behaviour to ascertain the torque required for breaking away and to subtract it from the torque required to achieve a specific speed,
in order in this way to be able to perform a determination of the property of the concrete in response to
both the signals indicative of speeds and torques and the breakaway behaviour.

13. Mixing device (1) according to the preceding claim, **characterized in that** the means (9) for determining at least one property of the concrete in response to signals indicative of speeds and torques is designed as a process computer which is designed for feeding in the digitized and conditioned speed-and torque-indicative signals.

## Revendications

1. Procédé de fabrication de béton au moyen d'un dispositif de malaxage, sur lequel
des signaux indiquant une vitesse de rotation et un couple sont détectés,
et une propriété de béton est déterminée en réponse à ceux-ci,
**caractérisé en ce que**
lors de la détermination de la propriété de béton, le comportement de décollage du dispositif est pris en compte,
**en ce que** le couple nécessaire au décollage est établi et soustrait du couple nécessaire pour obtenir une certaine vitesse de rotation.

2. Procédé de fabrication de béton haute performance et/ou de béton auto-plaçant selon la revendication précédente, de préférence avec un volume par charge issu du groupe comprenant : supérieur à 100 litres, supérieur à 200 litres, supérieur à 500 litres, supérieur à 1000 litres et/ou de préférence dans un dispositif de malaxage stationnaire ou transportable.

3. Procédé selon l'une quelconque des revendications précédentes, **caractérisé en ce qu'**un élément du groupe comprenant un malaxeur à un seul outil de malaxage, un malaxeur à un seul groupe d'outils de malaxage mobiles en combinaison, un turbo-malaxeur, un malaxeur à bac, un malaxeur planétaire, un malaxeur à une hélice ou à deux hélices est utilisé comme dispositif de malaxage, de préférence avec un engrenage issu du groupe comprenant un engrenage planétaire, un engrenage à vis sans fin et/ou une poulie à courroie entre l'arbre ou les arbres d'entraînement et un ou plusieurs moteurs d'entraînement.

4. Procédé selon l'une quelconque des revendications précédentes, **caractérisé en ce que** pour la mesure de signaux indiquant une vitesse de rotation et un couple,
un capteur de vitesse de rotation est prévu sur un élément parmi
un arbre malaxeur,
un arbre de transmission, et
un moteur d'entraînement,
et/ou des signaux indiquant un couple sont détectés
avant
ou après un engrenage,
de préférence par la détection
d'une puissance d'entraînement électrique alimentée
et/ou d'une fréquence de convertisseur pour l'entraînement d'arbre malaxeur.

5. Procédé selon l'une quelconque des revendications précédentes, **caractérisé en ce que** les signaux indiquant une vitesse de rotation et un couple sont détectés
sur au moins un élément parmi
un engrenage,
un moteur, et
un arbre/des arbres malaxeur(s),
et/ou
la détection comprend
l'alimentation
des signaux numérisés et conditionnés indiquant une vitesse de rotation et un couple
dans une unité de calculateur de processus.

6. Procédé selon l'une quelconque des revendications précédentes, **caractérisé en ce qu'**au moins un comportement au fluage du béton est déterminé comme propriété de béton.

7. Procédé selon l'une quelconque des revendications précédentes, **caractérisé en ce que** la détection des signaux indiquant une vitesse de rotation et un couple comprend la mesure d'une série d'au moins 2, de préférence d'au moins 3, de façon particulièrement préférée d'au moins 5 valeurs de mesure qui sont détectées pour un élément du groupe comprenant des vitesses de rotation jusqu'à 0,25 fois la vitesse de rotation maximale pendant le processus de malaxage ; des vitesses de rotation jusqu'à 0,25 fois la vitesse de rotation moyenne dans le temps pendant le processus de malaxage ; et **en ce qu'**à partir de la série d'au moins 2 ou d'au moins 3 ou d'au moins 5 valeurs de mesure, une interpolation vers la vitesse de rotation N = 0 est effectuée ; et/ou la détection des signaux indiquant une vitesse de rotation et un couple est effectuée sur le dispositif de manière intermittente par rapport au processus de malaxage, de préférence sous une sollicitation au cisaillement qui est au moins de 10 % inférieure à celle survenant au maximum lors du malaxage du mélange de béton, de préférence en moyenne, de manière particulièrement préférée sous une sollicitation au cisaillement d'un maximum de 25 % de celle survenant au malaxage.

8. Procédé selon l'une quelconque des revendications précédentes, **caractérisé en ce que** pour la détermination des signaux indiquant une vitesse de rotation et un couple sur le dispositif, le couple est augmenté progressivement, et ensuite on attend jusqu'à ce que la vitesse de rotation se soit stabilisée et une phase de plateau soit atteinte.

9. Procédé selon l'une quelconque des revendications précédentes, **caractérisé en ce que** le comportement de décollage est compensé par le fait que le comportement de décollage est détecté avec un dispositif vide ou substantiellement vide, et les signaux indiquant une vitesse de rotation et un couple sont corrigés par rapport à celui-ci lorsque le dispositif est plein, dans lequel, de préférence, lors de la détermination du comportement de décollage à vide, le dispositif vide ou substantiellement vide est rempli à moins de 10 % de son volume de chambre de malaxage, de plus grande préférence est rempli à moins de 5 % du volume, de plus grande préférence est rempli à moins de 1 % du volume, et de manière particulièrement préférée est rempli à moins de 0,5 % du volume.

10. Procédé selon l'une quelconque des revendications précédentes, dans lequel le comportement de décollage du dispositif est déterminé pour une pluralité de conditions de fonctionnement, en particulier en fonction de la température d'un ou de plusieurs composants, en particulier de la transmission et/ou d'une huile de transmission et/ou de la viscosité de lubrifiants, dans lequel de préférence un réseau de caractéristiques et/ou une table de consultation sont établis et une compensation sur le couple de décollage est effectuée en tenant compte des conditions de fonctionnement.

11. Procédé selon l'une quelconque des revendications précédentes, **caractérisé en ce que** la prise en compte du comportement de décollage du dispositif est effectuée en soustrayant le couple nécessaire au décollage de valeurs individuelles d'une série de valeurs de mesure qui sont enregistrées à différentes vitesses de rotation et/ou avec une entrée de couple différente, en particulier par soustraction d'une grandeur dérivée d'un réseau de caractéristiques ou d'une caractérisation de dispositif et/ou d'une dépendance thermique.

12. Dispositif de malaxage (1) destiné à exécuter un procédé selon l'une quelconque des revendications précédentes, comprenant
un moyen (7, 8) pour détecter des signaux indiquant des vitesses de rotation et des couples,
et
un moyen pour déterminer au moins une propriété de béton en réponse à ceux-ci,
**caractérisé en ce que**
le moyen (9) pour la détermination d'au moins une propriété de béton, en réponse à des signaux indiquant des vitesses de rotation et des couples,
en tant que moyen pour la détermination d'au moins une propriété de béton en réponse à des signaux indiquant des vitesses de rotation et des couples en tenant compte du comportement de décollage, est réalisé pour établir le couple nécessaire au décollage et pour le soustraire du couple nécessaire à l'obtention d'une certaine vitesse de rotation,
afin de pouvoir ainsi procéder à une détermination de la propriété de béton en réponse à la fois aux signaux indiquant des vitesses de rotation et des couples et au comportement de décollage.

13. Dispositif de malaxage (1) selon la revendication précédente, **caractérisé en ce que** le moyen (9) de détermination d'au moins une propriété de béton en réponse à des signaux indiquant des vitesses de rotation et des couples est réalisé sous forme de calculateur de processus qui est réalisé pour alimenter les signaux numérisés et conditionnés indiquant une vitesse de rotation et un couple.
